# EUROPÄISCHE PATENTSCHRIFT

(11) **EP 3 491 364 B1**
(45) Veröffentlichungstag und Bekanntmachung des Hinweises auf die Patenterteilung: **02.06.2021**
(21) Anmeldenummer: 17758285.5
(22) Anmeldetag: 26.07.2017
(51) Int. Cl.: G01N 21/65, C12Q 1/02, C12Q 1/18, G01J 3/44, G01N 33/569

(54) **KOMBINIERTES OPTISCH-SPEKTROSKOPISCHES VERFAHREN ZUR BESTIMMUNG VON MIKROBIELLEN ERREGERN**
COMBINED OPTICAL-SPECTROSCOPIC METHOD FOR IDENTIFYING MICROBIAL PATHOGENS
PROCÉDÉ COMBINÉ OPTIQUE-SPECTROSCOPIQUE POUR L'IDENTIFICATION D'AGENTS MICROBIENS

(30) Priorität: 26.07.2016 DE 102016113748
(43) Veröffentlichungstag der Anmeldung: 05.06.2019
(73) Patentinhaber: Institut für Photonische Technologien e.V., 07745 Jena (DE); Universitätsklinikum Jena, 07743 Jena (DE)
(72) Erfinder: NEUGEBAUER, Ute, 07743 Jena (DE); POPP, Jürgen, 07751 Jena-Kunitz (DE); STRANIK, Ondrej, 07743 Jena (DE); LÖFFLER, Bettina, 07749 Jena (DE)
(74) Vertreter: Maiwald Patent- und Rechtsanwaltsgesellschaft mbH
(86) Internationale Anmeldenummer: PCT/IB2017/054533
(87) Internationale Veröffentlichungsnummer: WO 2018/020433

(56) Entgegenhaltungen:
- US-A- 5 573 927
- US-A1- 2007 109 535
- US-A1- 2011 143 332
- US-A1- 2012 149 599
- US-A1- 2014 377 795
- US-A1- 2016 053 295
- Anonymous: "Combined Dielectrophoresis-Raman Setup for the Classification of Pathogens Recovered from the Urinary Tract - Analytical Chemistry (ACS Publications)", , 14. Oktober 2013 (2013-10-14), XP055425245, Gefunden im Internet: URL:http://pubs.acs.org/doi/10.1021/ac4021 616 [gefunden am 2017-11-15]
- K. MAQUELIN ET AL: "Prospective Study of the Performance of Vibrational Spectroscopies for Rapid Identification of Bacterial and Fungal Pathogens Recovered from Blood Cultures", JOURNAL OF CLINICAL MICROBIOLOGY, Bd. 41, Nr. 1, 1. Januar 2003 (2003-01-01) , Seiten 324-329, XP055345994, US ISSN: 0095-1137, DOI: 10.1128/JCM.41.1.324-329.2003

## Beschreibung

Die Erfindung betrifft Verfahren zur Bestimmung eines mikrobiellen Erregers. Außerdem betrifft die Erfindung Verfahren zur Bestimmung eines mikrobiellen Erregers und dessen Antiinfektivaresistenz. Insbesondere betrifft die Erfindung ein Verfahren zur Bestimmung eines Bakteriums und dessen Antibiotikaresistenz.

### Technologischer Hintergrund

Die derzeit etablierten Techniken zur phänotypischen Resistenztestung aus Patientenmaterial basieren auf mindestens zwei Kulturschritten, so dass das Analyseergebnis frühestens 48 Stunden nach Probenentnahme vorliegen kann. Der erste Kulturschritt in diesem Verfahren dient der Gewinnung einer reinen Bakterienkultur, der zweite beinhaltet die Resistenztestung selbst. Gängige Tests zur Resistenztestung sind der Agardiffusionstest, der E-Test und der Mikrodilutionstest. Die Identifizierung von Antibiotikaresistenzen mit Hilfe der Raman-Spektroskopie konnte für Vancomycin-resistente Enterokokken gezeigt werden (Schröder et al. Scientific Reports, 2015). Außerdem gibt es Algorithmen, die die Morphologie von Bakterien unter Einfluss von Antibiotika erfassen. Diese Algorithmen benötigen allerdings die vorherige Identifizierung der Bakterien und zeigten in Tests mit mehreren klinischen Isolaten noch eine Fehlerquote von fast 10% (Choi et al., Science Translational Medicine, 2015).

Automatisierte Assays benötigen größere Mengen an biologischem Material, das über eine Übernachtkultur gewonnen werden muss. Außerdem dauert die Diagnose häufig 8 bis 10 h, oder in manchen Fällen sogar länger. Neuere Ansätze, die auf einer genotypischen Identifizierung und Charakterisierung der Antibiotikaresistenz beruhen sind zwar deutlich schneller und können oft auch direkt aus dem Patientenmaterial durchgeführt werden, sie erfordern jedoch eine genaue Kenntnis der charakteristischen Nukleinsäuresequenzen und versagen daher oft bei der Identifizierung von schnell mutierenden multiresistenten Gram-negativen Erregern.

US 2011/143332 A1 offenbart ein Verfahren und eine Vorrichtung zur Herstellung eines Profils, das einen Mikroorganismus identifiziert, basierend auf oberflächenverstärkter Raman-Streuung (SERS).

Daher ist es mit den bisherigen Techniken nicht möglich, eine schnelle und zuverlässige Antibiotikaresistenz-Analyse von noch nicht identifizierten mikrobiellen Erregern wie Bakterien durchzuführen. Um die Antiinfektiva-Therapie, insbesondere die Antibiotika-Therapie, auf den Patienten und seinen mikrobiellen Erreger optimal zuzuschneiden, wären kürzere Bestimmungszeiten, insbesondere der Antibiotikaresistenzen, wünschenswert. Darüber hinaus könnte man dadurch den Einsatz von Breitbandantibiotika reduzieren sowie falsche Therapien bei unbekannten Resistenzen vermeiden.

### Zusammenfassung der Erfindung

Somit kann es als Aufgabe der Erfindung angesehen werden, ein verbessertes Verfahren zur Bestimmung eines mikrobiellen Erregers bereitzustellen. Insbesondere ist es die Aufgabe der Erfindung, ein verbessertes Verfahren zur gleichzeitigen Bestimmung eines mikrobiellen Erregers und dessen Antiinfektivaresistenz bereitzustellen. Im Speziellen kann es als eine Aufgabe der Erfindung angesehen werden, ein Verfahren zur gleichzeitigen Bestimmung eines Bakteriums und dessen Antibiotikaresistenz bereitzustellen.

Die Aufgabe der Erfindung wird mittels des Verfahrens des unabhängigen Anspruches 1 gelöst. Weitere Vorteile und Weiterbildungen sind in den Unteransprüchen angegeben.

Generell betrifft die vorliegende Erfindung ein Verfahren zur Bestimmung eines mikrobiellen Erregers umfassend die Schritte Raman-Spektroskopie einer Probe umfassend mindestens einen mikrobiellen Erreger und optische Detektion der Probe.

Die vorliegende Erfindung betrifft ein Verfahren gemäß dem beigefügtem Anspruch 1 zur Bestimmung eines mikrobiellen Erregers und dessen Antiinfektivaresistenz in einer Probe umfassend den mikrobiellen Erreger und ein Antiinfektivum.

Die optische Detektion kann durch Mikroskopie, holographische Detektion oder dynamische Lichtstreuung erfolgen. In bevorzugten Ausführungsformen erfolgt die optische Detektion durch Mikroskopie. Das mikroskopische Verfahren kann beispielsweise Durchlichtmikroskopie oder Fluoreszenzmikroskopie sein. Als bevorzugtes mikroskopisches Verfahren wird die Durchlichtmikroskopie eingesetzt. Bei der Bestimmung der Morphologie des mikrobiellen Erregers durch optische Detektion werden quantitative Morphologieparameter bestimmt. Entsprechend basiert die Bestimmung des mikrobiellen Erregers auf dem Raman-Spektrum und den quantitativen Morphologieparametern des mikrobiellen Erregers.

Durch die Kombination der optisch-morphologischen und Raman-spektroskopischen Bestimmung wird eine schnelle und zuverlässige Bestimmung des mikrobiellen Erregers ermöglicht. Die Identifizierung des mikrobiellen Erregers und ggf. dessen Antiinfektiva-resistenz erfolgt durch automatisierte Analyse, z.B. anhand von multivariable statistischen Methoden und Abgleich mit Datenbanken, die lokal oder zentral verfügbar sind.

Dies erlaubt in kurzer Zeit anhand einer geringen Anzahl von mikrobiellen Erregern die Bestimmung der mikrobiellen Erreger und deren Antibiotikaresistenz. Es kann also innerhalb von kürzester Zeit und einer geringen Erregeranzahl die Erregerart, das Resistenzlevel und/oder die Mindesthemmkonzentration eines Antiinfektivums für den (zu bestimmenden) mikrobiellen Erreger bestimmt werden. Anhand der gewonnen Informationen kann die Therapie gezielt auf den spezifischen Erreger abgestimmt werden.

Die Mindesthemmkonzentration ergibt sich als die Konzentration, bei der ein Schwellenwert (z. B. Bandenverhältnis in den Raman-Spektren oder bestimmtes Ausmaß der Morphologieveränderung oder bestimmte Reduktion des Bakterienwachstums im Vergleich zur unbehandelten Probe) gerade nicht erreicht bzw. noch nicht überschritten wird. Durch Vergleich der so bestimmten Mindesthemmkonzentration mit den Grenzwerten der Empfindlichkeit (z. B. nach EUCAST-Richtlinie) erfolgt die Einstufung der unbekannten, zu charakterisierenden mikrobiellen Erreger in die Resistenzlevel, wie z.B. sensibel, intermediär oder resistent.

Die Begriffe "Bestimmen", "Bestimmung" und "Identifizierung" sind in dieser Anmeldung austauschbar verwendbar.

Eine bevorzugte Ausführungsform betrifft ein Verfahren zur Bestimmung des mikrobiellen Erregers und dessen Antiinfektivaresistenz umfassend die Schritte Raman-Spektroskopie einer Probe umfassend mindestens einen mikrobiellen Erreger und ein Antiinfektivum und optische Detektion der Probe, und Bestimmung der Antiinfektivaresistenz basierend auf dem Raman-Spektrum und der Morphologie des mikrobiellen Erregers.

In einer spezifischen Ausführungsform umfasst das Verfahren ferner den Schritt:
- Bestimmen des Zellwachstums des mikrobiellen Erregers durch optische Detektion
wobei die Bestimmung der Antiinfektivaresistenz auf dem bestimmten Raman-Spektrum, der bestimmten Morphologie und dem bestimmten Zellwachstum des mikrobiellen Erregers basiert.

Bei der Bestimmung der Antiinfektivaresistenz des mikrobiellen Erregers kann ein erster Indikator für die Resistenz des mikrobiellen Erregers gegen das Antiinfektivum verwendet werden, wobei der erste Indikator für die Resistenz des mikrobiellen Erregers gegen das Antiinfektivum eine unveränderte Zellmorphologie des mikrobiellen Erregers im Vergleich zu Negativkontrolldaten ist. Ferner, kann bei der Bestimmung der Antiinfektivaresistenz des mikrobiellen Erregers ein zweiter Indikator für die Resistenz des mikrobiellen Erregers gegen das Antiinfektivum verwendet werden, wobei der zweite Indikator für die Resistenz des mikrobiellen Erregers gegen das Antiinfektivum ein unverändertes oder ein für einen bestimmten Resistenzmechanismus charakteristisch verändertes Raman-Spektrum des mikrobiellen Erregers im Vergleich zu Negativkontrolldaten ist. Außerdem kann bei der Bestimmung der Antiinfektivaresistenz des mikrobiellen Erregers ein dritter Indikator für die Resistenz des mikrobiellen Erregers gegen das Antiinfektivum verwendet werden, wobei der dritte Indikator für die Resistenz des mikrobiellen Erregers gegen das Antiinfektivum ein unverändertes oder geringfügig unverändertes Zellwachstum des mikrobiellen Erregers im Vergleich zu Negativkontrolldaten ist.

Die Negativkontrolldaten können Daten von einer Probe mit dem mikrobiellen Erreger ohne Antiinfektivum sein. Die Daten dieser Probe können bereits vorliegen, z.B. als Daten einer Probe, die bereits zu einem früheren Zeitpunkt gemäß dem vorliegenden Verfahren analysiert wurde. Alternativ kann die Probe mit dem mikrobiellen Erreger ohne Antiinfektivum parallel zur Probe mit dem Antiinfektivum analysiert werden. In einer anderen Ausführungsform können die Negativkontrolldaten von derselben Probe, bevor sie in Kontakt mit dem Antiinfektivum gekommen ist, stammen.

Im Speziellen betrifft die vorliegende Anmeldung Verfahren zur Bestimmung eines mikrobiellen Erregers und dessen Antiinfektivaresistenz in einer Probe umfassend den mikrobiellen Erreger und ein Antiinfektivum, umfassend die Schritte: Kultivierung der Probe umfassend mindestens einen mikrobiellen Erreger und ein Antiinfektivum, Bestimmen des Raman-Spektrums des mikrobiellen Erregers durch Raman-Spektroskopie der Probe unter mindestens zwei unterschiedlichen Antiinfektivum-Bedingungen und Bestimmen der Morphologie des mikrobiellen Erregers durch optische Detektion der Probe unter mindestens zwei unterschiedlichen Antiinfektivum-Bedingungen.

Insbesondere betrifft die vorliegende Anmeldung ein Verfahren zur Bestimmung eines mikrobiellen Erregers und dessen Antiinfektivaresistenz umfassend die Schritte: Kultivieren einer Probe umfassend mindestens einen mikrobiellen Erreger und ein Antiinfektivum; Raman-Spektroskopie der Probe zu mindestens zwei Zeitpunkten während der Kultivierung der Probe und optische Detektion der Probe zu mindestens zwei Zeitpunkten während der Kultivierung der Probe.

"Unter mindestens zwei unterschiedlichen Antiinfektivum-Bedingungen" bedeutet in Zusammenhang der Erfindung, dass die Probe entweder einer unterschiedlichen Antiinfektivum-Konzentration und/oder einer unterschiedlichen Wirkdauer des Antiinfektivums ausgesetzt ist. In einer Ausführungsform kann die Probe in zwei oder mehrere Aliquote (Teile) aufgeteilt werden, wobei jedem Aliquot eine andere Konzentration des Antiinfektivums zugesetzt wird. Dies schließt ein, dass einer Einheit der Probe kein Antiinfektivum zugesetzt wird, während einer anderen Einheit eine bestimmte Konzentration des Antiinfektivums zugesetzt wird. Nach der Einwirkzeit des Antiinfetivums kann die Messung zu einem Zeitpunkt stattfinden. Die Wirkdauer oder Einwirkzeit des Antiinfektivums kann 5 min, 10 min, 15 min, 20 min, 30 min, 40 min, 45 min, 50 min, 60 min, 70 min, 80 min, 90 min, 120 min oder länger betragen. Dem Fachmann sind die Wirkdauern der Antiinfektiva bekannt.

In einer anderen Ausführungsform werden das Raman-Spektrum und die Morphologie der Probe zu mindestens zwei Zeitpunkten bestimmt. Wobei mindestens an einem früheren Zeitpunkt, zu dem das Antiinfektivum noch nicht zugegeben wurde oder zu dem das Antiinfektivum seine Wirkung noch nicht entfalten konnte, und mindestens an einem späteren Zeitpunkt, zu dem das Antiinfektivum seine Wirkung entfalten konnte, gemessen wird.

In einer Ausführungsform wird zum früheren Zeitpunkt wird das Antiinfektivum noch nicht zugegeben. Alternativ wird das Antiinfektivum zum früheren Zeitpunkt zugegeben und hat zum früheren Zeitpunkt eine kürzere Einwirkdauer auf die Probe als zum späteren Zeitpunkt. Der frühere Zeitpunkt zur Bestimmung des Raman-Spektrums und der Morphologie der Probe kann beispielsweise 5 min nach Antiinfektivumzugabe oder früher, bevorzugt 3 min nach Antinfektivumzugabe oder früher, mehr bevorzugt 2 min nach Antiinfektivumzugabe oder früher, besonders bevorzugt 1 min nach Antiinfektivumzugabe oder früher oder vor der Antiinfektivumzugabe sein. Der spätere Zeitpunkt kann beispielsweise nach ca. 10 min, 15 min, 20 min, 30 min 40 min, 45 min, 50 min, 60 min, 70 min, 80 min, 90 min, 120 min oder länger nach der Antiinfektivumzugabe sein.
Durch die Kombination von optisch-morphologischen und Raman-spektroskopischen Analysen wird die gleichzeitige Identifizierung des mikrobiellen Erregers als auch die Antiinfektivaresistenz-Bestimmung in einem Test ermöglicht. Außerdem wird durch das kombinierte Verfahren eine schnellere und zugleich empfindlichere Resistenztestung ermöglicht. Das Verfahren ist einfach zu parallelisieren und kann daher in der Routinediagnostik zum Testen von mehreren Antiinfektiva (Resistogramm), insbesondere Antibiotika, eingesetzt werden. Für dieses Verfahren sind geringe Mengen von mikrobiellen Erregern, wie z.B. 100-1000 Bakterien pro Test, ausreichend. Daher können zeitaufwändige Kultivierungs- und Isolierungsschritte vermieden werden.

Die Kombination von optischer Detektion und Raman-Spektroskopie dient dabei nicht nur zum Auffinden der Mikroorganismen, sondern beide Techniken liefern wichtige Informationen für die Bestimmung des mikrobiellen Erregers und/oder seiner Antibiotikaresistenz. D.h., dass nicht nur die Daten der Raman-Spektroskopie, sondern auch die durch die optische Detektion bestimmten morphologischen Parameter zur Bestimmung des mikrobiellen Erregers und/oder seiner Antibiotikaresistenz verwendet werden. Die optische Detektion wird also nicht nur zur Bestimmung der lokalen Position des mikrobiellen Erregers verwendet.

Im Speziellen betrifft die Anmeldung Verfahren zur Bestimmung eines mikrobiellen Erregers und dessen Antiinfektivaresistenz in einer Probe umfassend den mikrobiellen Erreger und ein Antiinfektivum, umfassend die Schritte: Kultivierung der Probe umfassend mindestens einen mikrobiellen Erreger und ein Antiinfektivum, Bestimmen des Raman-Spektrums des mikrobiellen Erregers durch Raman-Spektroskopie der Probe unter mindestens zwei unterschiedlichen Antiinfektivum-Bedingungen, Bestimmen der Morphologie des mikrobiellen Erregers durch optische Detektion der Probe unter mindestens zwei unterschiedlichen Antiinfektivum-Bedingungen, Bestimmen des mikrobiellen Erregers basierend auf dem Raman-Spektrum und der Morphologie des mikrobiellen Erregers, Bestimmen der Antiinfektivaresistenz des mikrobiellen Erregers basierend auf dem Raman-Spektrum und der Morphologie des mikrobiellen Erregers.

Insbesondere betrifft die Anmeldung Verfahren zur Bestimmung eines Bakteriums und dessen Antibiotikaresistenz umfassend die Schritte: Kultivieren einer Probe umfassend mindestens ein Bakterium und ein Antibiotikum; Raman-Spektroskopie der Probe zu mindestens zwei Zeitpunkten während der Kultivierung der Probe, und optische Detektion der Probe zu mindestens zwei Zeitpunkten während der Kultivierung der Probe. Die vorliegende Anmeldung eignet sich besonders zur Identifizierung eines multiresistenten Gram-negativen Bakteriums und dessen Antibiotikaresistenz. Daher ist die vorliegende Anmeldung insbesondere den molekularbiologischen Methoden, die auf der Bestimmung von bestimmten Resistenzgenen beruhen, überlegen. Denn mit diesen Methoden ist es bisher nicht möglich, die derzeit vermehrt auftretenden multiresistenten Gram-negativen Erreger (MRGN) mit ihrer hohen genetischen Variabilität sicher zu erfassen. Die vorliegende Anmeldung ist auch dazu geeignet, einen Pilz zu bestimmen sowie dessen Antimykotikaresistenz festzustellen.

Typischerweise, findet die Raman-Spektroskopie auf Einzelzellniveau statt. Das heißt, dass nach einer Weitfeldaufnahme, in dieser die einzelnen mikrobiellen Erreger durch Bilderkennungsalgorithmen detektiert werden, die Raman-Spektroskopie jeweils an einzelnen mikrobiellen Erregern durchgeführt wird. Alternativ kann die Raman-Spektroskopie an mehreren Bakterien im Bildfeld gleichzeitig stattfinden. Zur Identifizierung von Mischinfektionen können dazu Entmischungsalgorithmen herangezogen werden.

Die optische Detektion kann sowohl im Weitfeld zur Bestimmung der Anzahl der mikrobiellen Erreger in der Probe eingesetzt werden als auch auf Einzelzellniveau, um die Zellmorphologie des mikrobiellen Erregers zu bestimmen. Um die Morphologie des mikrobiellen Erregers sicher in der Flüssigkeit erfassen zu können, können inhomogene elektrische Felder zu Hilfe genommen werden, die eine Ausrichtung der dielektrischen Mikroorgnismen im E-Feld bewirken. Durch diese Ausrichtung wird die zufällige Anordnung der Mikroorgnismen verringert. Insbesondere bei nicht-runden Erregern (wie z. B. Stäbchen) ist dies von großem Interesse und vereinfacht die Datenanalyse und verringert die Anzahl der für eine bestimmte Genauigkeit mindestens optisch erfassten Mikroorganismen. In einer Ausführungsform wird das Raman-Spektrum zu unterschiedlichen Zeitpunkten der Kultivierung gemessen und damit Informationen über den biochemischen Fingerabdruck des mikrobiellen Erregers gewonnen. Dadurch kann die Wirkung des Antiinfektivums bestimmt werden, d.h. die erfolgreiche Hemmung/Tötung durch das Antiinfektivum oder Induktion von verschiedenen Resistenzmechanismen). Somit erlaubt die Kombination des Raman-spektroskopischen Verfahrens und der optisch-morphologischen Analyse die effektive Bestimmung des Mikroorganismus (Identifizierung), die Bestimmung der Anzahl der mikrobiellen Erreger in der Probe, die Bestimmung, ob eine Resistenz vorliegt sowie den Wirkmechanismus der Antibiotikaresistenz.
Im Stand der Technik gibt es kein Verfahren, das diese Informationen innerhalb von kurzer Zeit (wenige Stunden) aus geringstem Probenmaterial (wenige Bakterien in Suspension) bereitstellen kann.

In spezifischen Ausführungsformen kann das Verfahren zusätzlich mit einer Kontrollprobe durchgeführt werden, die den mindestens einen mikrobiellen Erreger, jedoch kein Antiinfektivum, z.B. Antibiotikum, enthält. Natürlich kann das Verfahren auch mit mehreren Proben durchgeführt werden, die den mindestens einen mikrobiellen Erreger und jeweils ein anderes Antiinfektivum, wie z.B. ein Antibiotikum enthalten. Alternativ oder zusätzlich kann das Verfahren in mehreren Proben durchgeführt werden, die jeweils den mindestens einen mikrobiellen Erreger und ein Antiinfektivum in jeweils einer unterschiedlichen Konzentration enthalten, um so u.a. quantitative Aussagen zur minimalen Hemmkonzentration (MHK) gewinnen zu können. Außerdem kann als weitere Kontrollprobe ein Antibiotikasensitiver Teststamm verwendet werden. Den Proben mit dem Antibiotika-sensitiven Teststamm werden die gleichen Antibiotikaverbindungen in den gleichen Konzentrationen zugesetzt wie in den Proben mit dem zu untersuchenden mikrobiellen Erreger.

Üblicherweise findet die optische Detektion zu Beginn der Kultivierung und in Zeitintervallen von höchstens 60 Minuten, bevorzugt höchstens 30 Minuten, mehr bevorzugt höchstens 15 Minuten, besonders bevorzugt höchstens 5 Minuten statt.

Die Raman-Spektroskopie findet zu Beginn der Kultivierung und in Zeitintervallen von höchsten 60 Minuten, bevorzugt höchstens 30 Minuten statt.

Mit Hilfe des Verfahrens der Erfindung kann die Bestimmung des mikrobiellen Erregers und dessen Antiinfektivaresistenzen innerhalb von 180 Minuten, bevorzugt 150 Minuten, noch mehr bevorzug weniger als 120 Minuten, am meisten bevorzugt weniger als 60 Minuten abgeschlossen werden.

Typischerweise bezieht sich das Verfahren auf die Identifizierung von pathogenen mikrobiellen Erregern. Daher wird die Probe aus einem Individuum, bevorzugt einem Säugetier, besonders bevorzugt einem Menschen entnommen. Die Probe kann beispielsweise eine Körperflüssigkeit wie eine Urinprobe oder eine in ein Medium überführte Mikrokolonie sein.

Für die Verfahren gemäß der Erfindung sind lediglich 50 bis 1000, bevorzugt 100 bis 1000 mikrobielle Erreger in einer Probe notwendig. Typischerweise enthält eine Probe 5 x 10¹ bis 1 x 10⁵ bevorzugt 1 x 10² bis 1 x 10⁵, mehr bevorzugt 5 x 10² bis 1 x 10⁵ mikrobielle Erreger. In besonders bevorzugten Ausführungsformen enthält die Probe 1 x 10³ bis 1 x 10⁴ mikrobielle Erreger.

In einer Ausführungsform wird in der Probenkammer ein elektrisches inhomogenes Feld erzeugt.

Ein weiterer Aspekt der Anmeldung betrifft ein System zur Bestimmung eines mikrobiellen Erregers umfassend:
- Raman-Spektrometer zur Bereitstellung eines Raman-Spektrums des mikrobiellen Erregers,
- Vorrichtung zur optischen Detektion des mikrobiellen Erregers zur Bereitstellung von optischen Daten des mikrobiellen Erregers,
wobei das System zur Bestimmung der Morphologie des mikrobiellen Erregers durch Analyse der optischen Daten und zur Bestimmung des mikrobiellen Erregers basierend auf einer Kombination des Raman-Spektrums und der morphologischen Daten des mikrobiellen Erregers ausgeführt ist.

In einer spezifischen Ausführungsform ist das System ferner zur Bestimmung der Antiinfektivaresistenz auf einer Kombination des bestimmten Raman-Spektrums und der bestimmten Morphologie des mikrobiellen Erregers ausgeführt. Unter Verwendung verschiedener Antiinfektiva-Konzentrationen lässt sich so auch die minimale Hemmkonzentration des Erregers ermitteln. Dazu können Antiinfektivakonzentrationen um den klinischen "Breakpoint" (z. B. aus EUCAST-Richtlinien) eingesetzt werden.

In einer Ausführungsform umfasst das System zusätzlich eine Vorrichtung, die ein inhomogenes elektrisches Feld in einer zu bestimmenden Probe erzeugen kann. Die Vorrichtung umfasst beispielsweise Elektroden. Die Feldstärke kann dabei individuell an die Probe, die Kammer- und die Elektrodengeometrie angepasst werden und sollte zwischen 2 und 60 V betragen. Wenn keine aktive Bewegung zum Wärmeabtransport stattfindet und das Probenkammermaterial nur schlecht Wärmeleitend ist, so sollten 16 V nicht überschritten werden, um Heizeffekte zu vermeiden.

Ein weiterer Aspekt der Anmeldung bezieht sich auf ein Programmelement zur Bestimmung eines Erregers, das, wenn es auf einem Prozessor ausgeführt wird, den Prozessor anleitet, die folgenden Schritte durchzuführen:
- Bestimmen einer Morphologie des mikrobiellen Erregers basierend auf den Daten einer Vorrichtung zur optischen Detektion des Erregers, und
- Bestimmen des mikrobiellen Erregers basierend auf einem Raman-Spektrum des mikrobiellen Erregers und der Morphologie des mikrobiellen Erregers.

In einer Ausführungsform leitet das Programmelement den Prozessor an ferner den folgenden Schritt auszuführen:
- Bestimmen der Antiinfektivaresistenz des mikrobiellen Erregers basierend auf dem Raman-Spektrum und der Morphologie des mikrobiellen Erregers.

Ein weiterer Aspekt der Erfindung bezieht sich auf ein computerlesbares Medium, auf dem ein Programmelement gespeichert ist, das, wenn es auf einem Prozessor ausgeführt wird, den Prozessor anleitet, die folgenden Schritte durchzuführen:
- Bestimmen eines Raman-Spektrums des mikrobiellen Erregers basierend auf spektroskopischen Daten eines Raman-Spektrometers,
- Bestimmen einer Morphologie des mikrobiellen Erregers basierend auf den Daten einer Vorrichtung zur optischen Detektion des Erregers, und
- Bestimmen des mikrobiellen Erregers basierend auf dem Raman-Spektrum und der Morphologie des mikrobiellen Erregers.

In einer Ausführungsform kann das computerlesbare Medium den Prozessor anleiten ferner den folgenden Schritt durchzuführen: Bestimmen der Antiinfektivaresistenz des mikrobiellen Erregers basierend auf dem Raman-Spektrum und der Morphologie des mikrobiellen Erregers.

Zur Vereinfachung des optischen Erfassens der Morphologie kann der Probenraum Elektroden enthalten, die ein inhomogenes elektrisches Feld erzeugen und so eine Kraft auf die in der Probe enthaltenen Mikroorganismen ausüben. Durch diese Kraft werden mikrobiellen Erreger in eine bestimmte Orientierung getrieben, und/oder auf bestimmten Regionen des Messchips angereichert. Das Antiinfektivum kann einen Einfluss auf die Polarisierbarkeit des Mikroorganismus haben. Die Einwirkung eines elektrischen Feldes auf die Probe kann somit nicht nur zur Anreicherung im Detektionsfeld sondern auch zur Differenzierung des Mikroorganismus beitragen.

### Beschreibung bevorzugter Ausführungsformen

Es sei darauf hingewiesen, dass "umfassend" keine anderen Elemente oder Schritte ausschließt und "eine" oder "ein" keine Vielzahl ausschließt. Ferner sei darauf hingewiesen, dass Merkmale oder Schritte, die im Verweis auf eines der Ausführungsbeispiele beschrieben werden, auch in Kombination mit anderen Merkmalen oder Schritten anderer beschriebener Ausführungsbeispiele verwendet werden können.

Insbesondere sei daraufhingewiesen, dass sich die Verfahren zur Bestimmung eines mikrobiellen Erregers, auch auf die Bestimmung mehrerer mikrobieller Erreger beziehen, d.h. dass beispielsweise in einer Probe mehrere unterschiedliche Erreger enthalten sind oder mehrere Proben mit unterschiedlichen Erregern parallel untersucht werden. Ebenso können einer Probe mehr als ein Antiinfektivum zugesetzt werden oder mehrere Proben mit Antiinfektiva parallel untersucht werden. Natürlich sind auch Kombinationen von mehreren Erregern und Antiinfektiva beabsichtigt.

Die vorliegende Anmeldung betrifft ein Verfahren zur Bestimmung des mikrobiellen Erregers umfassend die Schritte Raman-Spektroskopie einer Probe umfassend mindestens einen mikrobiellen Erreger und optische Detektion der Probe.

Eine bevorzugte Ausführungsform betrifft ein Verfahren zur Bestimmung des mikrobiellen Erregers und dessen Antiinfektivaresistenz umfassend die Schritte Raman-Spektroskopie einer Probe umfassend mindestens einen mikrobiellen Erreger und ein Antiinfektivum und optische Detektion der Probe.

Eine spezifische Ausführungsform bezieht sich auf ein Verfahren zur Bestimmung des mikrobiellen Erregers und dessen Antiinfektivaresistenzen, umfassend die Schritte:
- Kultivierung einer Probe umfassend mindestens einen mikrobiellen Erreger und ein Antiinfektivum;
- Raman-Spektroskopie der Probe zu mindestens zwei Zeitpunkten während der Kultivierung der Probe;
- optische Detektion der Probe zu mindestens zwei Zeitpunkten während der Kultivierung der Probe;

Besonders bevorzugte Ausführungsformen beziehen sich auf Verfahren zur Bestimmung eines mikrobiellen Erregers und dessen Antiinfektivaresistenz umfassend die Schritte:
- Kultivierung einer Probe umfassend mindestens einen mikrobiellen Erreger und ein Antiinfektivum;
- Bestimmen des Raman-Spektrums des mikrobiellen Erregers durch Raman-Spektroskopie zu mindestens zwei Zeitpunkten während der Kultivierung der Probe;
- Bestimmen der Morphologie des mikrobiellen Erregers durch optische Detektion zu mindestens zwei Zeitpunkten während der Kultivierung der Probe;
- Bestimmen des mikrobiellen Erregers und dessen Antiinfektivaresistenz basierend auf dem Raman-Spektrum und der Morphologie des mikrobiellen Erregers.

Eine weitere Ausführungsform bezieht sich auf Verfahren zur Bestimmung eines mikrobiellen Erregers und dessen Antiinfektivaresistenz umfassend die Schritte:
- Aufteilung der Probe umfassend mindestens einen mikrobiellen Erreger in mindesten zwei Aliquote;
- Versetzen der einzelnen Aliquote mit unterschiedlichen Konzentrationen des Antiinfektivums;
- Kultivierung der Aliquote;
- Bestimmen des Raman-Spektrums des mikrobiellen Erregers durch Raman-Spektroskopie;
- Bestimmen der Morphologie des mikrobiellen Erregers durch optische Detektion;
- Bestimmen des mikrobiellen Erregers und dessen Antiinfektivaresistenz basierend auf dem Raman-Spektrum und der Morphologie des mikrobiellen Erregers.

Typischer Weise findet das Bestimmen des Raman-Spektrums und das Bestimmen der Morphologie nach einer Einwirkzeit des Antiinfektivum statt.

Bevorzugt werden die Konzentrationen verwendet, die im Bereich des Grenzwertes für Sensitivität und Resistenz sind. Hierzu kann sich der Fachmann beispielsweise an den Schwellwerten (breakpoints) der Breakpointtabelle des *European Commitee on Antimicrobial Susceptibility Testing* (EUCAST) orientieren (http://eucast.org/clinical_breakpoints/). Die eingesetzte Konzentration kann beispielsweise der 0,1-fache, 0,2-fache, 0,3-fache, 0,4-fache, 0,5-fache, 0,6-fache, 0,7-fache, 0,8-fache, 0,9-fache, 1-fache, 2-fache, 4-fache, 8-fache oder höher-fache Wert des EUCAST-Schwellwertes sein. In einer Ausführungsform ist die eingesetzte Konzentration der 4-fache Wert des EUCAST-Schwellwertes.

Als interne Qualitätskontrolle kann zusätzlich die Proben zum Zeitpunkt vor der Zugabe des Antiinfektivums oder direkt nach Zugabe des Antiinfektivums ebenfalls mit Hilfe der Raman-Spektroskopie und der optischen Analyse untersucht werden. Der Begriff "mikrobielle Erreger" umfasst Mikroorganismen, wie z.B. Bakterien, Archaeen und Pilze. Insbesondere bezieht sich der Begriff auf Mikroorganismen, die pathogen sind für Tiere, bevorzugt Säugetiere, besonders bevorzugt Menschen.

In bevorzugten Ausführungsformen ist das Bakterium ein multiresistentes gramnegatives Bakterium. Beispiele für multiresistente gramnegative Bakterien sind Enterobacteriaceae, z.B. *Escherichia coli, Klebsiella pneumoniae, Klebsiella oxytoca, Proteus spp., Enterobacter spp., Pseudomonas aeruginosa* und *Acinetobacter baumannii.*

Der Begriff "Antiinfektivum" umfasst Verbindungen, die gegen Mikroorganismen wirken, d.h. die Mikroorganismen abtöten oder deren Wachstum hemmen. Der Begriff umfasst z.B. Antibiotika (gegen Bakterien), Virostatika (gegen Viren), Antimykotika (gegen Pilze) und Anthelminthika (gegen Würmer). Insbesondere umfasst der Begriff "Antiinfektivum" Antibiotika und Antimykotika. Die Antiinfektiva und Antibiotika umfassen u.a. β-Lactame, Glykopeptide, Polyketide, Makrolid-Antibiotika, Aminoglycosid-Antibiotika, Polypeptid-Antibiotika, Chinolone (und Fluorochinolone) und Sulfonamide. Antibiotika wirken beispielsweise durch Hemmung der Zellwandsynthese (z.B. Penicillin), Hemmung der Proteinbiosynthese (z.B. Kanamycin, Neomycin und Chloramphenicol), Hemmung der korrekten Nucleinsäurepolymerisation (z.B. Rifampicin und Ciprofloxacin).

Daher eignen sich die Verfahren der Erfindung besonders zur Bestimmung von Bakterien und deren Antibiotikaresistenz. Dabei wird eine Probe, die mindestens ein Bakterium und ein Antibiotikum umfasst der Raman-Spektroskopie und der optischen Detektion unterzogen. Außerdem eignen sich die Verfahren der Erfindung zur Bestimmung von Pilzen und deren Antimykotikaresistenz, wobei eine Probe, die mindestens einen Pilz und ein Antimykotikum umfasst, der Raman-Spektroskopie und der optischen Detektion unterzogen wird.

Ein antiinfektivaresistenter Mikroorganismus ist fähig, die wachstumshemmende oder tötende Wirkung abzuschwächen oder völlig zu neutralisieren. Dazu kann er verschiedene Strategien verwenden, z. B. die Ziel struktur des Antiinfektiviums modifizieren, so dass der Wirkstoff nicht mehr binden kann, Enzyme synthetisieren, die das Antiinfektivum abbauen und unwirksam machen, alternative Proteine synthetisieren, die die Funktion des durch das Antiinfektivum gehemmten Proteins übernehmen oder alternative Stoffwechselwege erschließen, das gehemmte Molekül in einem so großen Überschuss erzeugen, dass die vorhandenen Antiinfektivakonzentrationen nur einen Teil der Zielstrukturen blockieren können und der Rest noch vorliegt, Veränderungen in der Membranstruktur, so dass die Substanzen gar nicht erst in die Zelle gelangen (reduzierte Aufnahme) oder aktiv wieder herausgepumpt werden können (z. B. Efflux-Pumpen). Alle diese Veränderungen führen dazu, dass der Mikroorganismus trotz Anwesenheit des Antiinfektiviums weiterwachsen kann (Resistenz), es finden jedoch z. T. morphologische als auch biochemische Veränderungen statt, die optisch als auch Raman-Spektroskopisch erfasst werden können. Die oben genannten Resistenzmechanismen können sowohl einzeln als auch in Kombination auftreten. Durch Verwendung von elektrischen Feldern können die Veränderungen u.U. leichter sichtbar gemacht werden.

Bevorzugte Ausführungsformen beziehen sich auf Verfahren zur Bestimmung eines Bakteriums und dessen Antibiotikaresistenz umfassend die Schritte:
- Kultivierung einer Probe umfassend mindestens ein Bakterium und ein Antibiotikum;
- Raman-Spektroskopie der Probe zu mindestens zwei Zeitpunkten während der Kultivierung der Probe;
- optische Detektion der Probe zu mindestens zwei Zeitpunkten während der Kultivierung der Probe.

Besonders bevorzugte Ausführungsformen beziehen sich auf Verfahren zur Bestimmung eines Bakteriums und dessen Antibiotikaresistenz umfassend die Schritte:
- Kultivierung einer Probe umfassend mindestens ein Bakterium und ein Antibiotikum;
- Bestimmen des Raman-Spektrums des Bakteriums durch Raman-Spektroskopie zu mindestens zwei Zeitpunkten während der Kultivierung der Probe;
- Bestimmen der Morphologie des Bakteriums durch optische Detektion zu mindestens zwei Zeitpunkten während der Kultivierung der Probe;
- Bestimmen des Bakteriums und dessen Antibiotikaresistenz basierend auf dem Raman-Spektrum und der Morphologie des Bakteriums.

Alternative Ausführungsformen beziehen sich auf Verfahren zur Bestimmung eines Pilzes und dessen Antimykotikaresistenz umfassend die Schritte:
- Kultivierung einer Probe umfassend mindestens einen Pilz und ein Antimykotikum;
- Raman-Spektroskopie der Probe zu mindestens zwei Zeitpunkten während der Kultivierung der Probe;
- optische Detektion der Probe zu mindestens zwei Zeitpunkten während der Kultivierung der Probe.

Besonders bevorzugte Ausführungsformen beziehen sich auf Verfahren zur Bestimmung eines Pilzes und dessen Antimykotikaresistenz umfassend die Schritte:
- Kultivierung einer Probe umfassend mindestens einen Pilz und ein Antimykotikum;
- Bestimmen des Raman-Spektrums des Pilzes durch Raman-Spektroskopie zu mindestens zwei Zeitpunkten während der Kultivierung der Probe;
- Bestimmen der Morphologie des Pilzes durch optische Detektion zu mindestens zwei Zeitpunkten während der Kultivierung der Probe;
- Bestimmen des Pilzes und dessen Antimykotikaresistenz basierend auf dem Raman-Spektrum und der Morphologie des Pilzes.

Die Zeitintervalle zwischen den mindestens zwei Zeitpunkten werden an anderer Stelle definiert. In besonderen Ausführungsformen ist mindestens zum ersten Zeitpunkt (an dem die optische Detektion und/oder Raman-Spektroskopie stattfindet) die Probe noch nicht mit dem Antiinfektivum versetzt. Die Zugabe des Antiinfektivum erfolgt vor dem nächsten Zeitpunkt, beispielsweise dem zweiten Zeitpunkt (an dem die optische Detektion und/oder Raman-Spektroskopie stattfindet).

Eine unveränderte Zellmorphologie des mikrobiellen Erregers im Vergleich zu Negativkontrolldaten kann ein Indikator für eine Resistenz des mikrobiellen Erregers gegen das Antiinfektivum sein. Darüber hinaus kann ein unverändertes Zellwachstum des mikrobiellen Erregers im Vergleich zu Negativkontrolldaten ein Indikator für eine Resistenz des mikrobiellen Erregers gegen das Antiinfektivum sein. Außerdem, kann eine unverändertes oder ein für einen bestimmten Resistenzmechanismus charakteristisch verändertes Raman-Spektrum des mikrobiellen Erregers im Vergleich zu Negativkontrolldaten ein Indikator für die Resistenz des mikrobiellen Erregers gegen das Antiinfektivum sein. In anderen Worten, kann beim Bestimmen der Antiinfektivaresistenz des mikrobiellen Erregers ein erster Indikator für die Resistenz des mikrobiellen Erregers gegen das Antiinfektivum verwendet werden, wobei der erste Indikator für die Resistenz des mikrobiellen Erregers gegen das Antiinfektivum eine unveränderte oder für den Resistenzmechanismus charakteristische Veränderung der Zellmorphologie des mikrobiellen Erregers im Vergleich zu Negativkontrolldaten ist. Außerdem, kann bei der Bestimmung der Antiinfektivaresistenz des mikrobiellen Erregers ein zweiter Indikator für die Resistenz des mikrobiellen Erregers, gegen das Antiinfektivum verwendet werden, wobei der zweite Indikator für die Resistenz des mikrobiellen Erregers gegen das Antiinfektivum ein unverändertes oder ein für einen bestimmten Resistenzmechanismus charakteristisch verändertes Raman-Spektrum des mikrobiellen Erregers im Vergleich zu Negativkontrolldaten ist. Der Begriff "ein für einen bestimmten Resistenzmechanismus charakteristisch verändertes Raman-Spektrum" umfasst charakteristische spektrale Veränderungen, die dem Fachmann bekannt sind. Bei bestimmten Resistenzmechanismen kann eine veränderte Aminosäurezusammensetzung des Bakteriums detektiert werden (z.B. bei VanA, VanB, VanC, VanD, VanE, VanG, insbesondere VanA, VanB u.a. bei Vancomycin-Resistenz), bei anderen Resistenzmechanismen kann eine erhöhte Produktion bestimmter Enzyme oder der durch diese Enzyme gespaltenen Stoffen detektiert werden, wie z. B. bei einer β-Laktam-Resistenz eine erhöhte Produktion von β-Laktamasen oder eine verstärkte Hydrolyse des β-Laktamrings der eingesetzten β-Laktam-Antibiotika.

Außerdem kann bei dem Bestimmen der Antiinfektivaresistenz des mikrobiellen Erregers ein dritter Indikator für die Resistenz des mikrobiellen Erregers gegen das Antiinfektivum verwendet werden, wobei der dritte Indikator für die Resistenz des mikrobiellen Erregers gegen das Antiinfektivum ein unverändertes Zellwachstum des mikrobiellen Erregers im Vergleich zu Negativkontrolldaten ist.

Der Begriff "unverändertes Wachstum", wie er hier verwendet wird, umfasst auch schwach verringertes Zellwachstum. Das heißt, dass das Zellwachstum um weniger als 10%, weniger als 5%, weniger als 3% oder weniger als 1 % verringert ist im Vergleich zu Negativkontrolldaten. Das Zellwachstum wird in der Regel durch die Beobachtung der Zellzahl über die Zeit, d.h. über die Wachstumsrate bestimmt. Das Zellwachstum kann beispielsweise auch morphologisch detektiert werden, z.B. durch Zählen der Zellen, die eine für die Zellteilung typische Morphologie aufweisen. Umgekehrt, kann eine veränderte Zellmorphologie des mikrobiellen Erregers im Vergleich zu Negativkontrolldaten ein Indikator für eine Sensitivität des mikrobiellen Erregers gegen das Antiinfektivum sein. Insbesondere sind Veränderungen der Zellmorphologie zu berücksichtigen, die für die Sensitivität des mikrobiellen Erregers charakteristisch sind. In bestimmten Ausführungsformen können daher geringfügige initiale Formveränderungen vernachlässigt werden. Dabei sind die morphologischen Änderungen oft charakteristisch für bestimmte Antibiotikaklassen. Darüber hinaus, kann ein verändertes Zellwachstum des mikrobiellen Erregers im Vergleich zu Negativkontrolldaten ein Indikator für eine Sensitivität des mikrobiellen Erregers gegen das Antiinfektivum sein. Außerdem, kann ein für die Sensitivität gegen das Antiinfektivum charakteristisch verändertes Raman-Spektrum des mikrobiellen Erregers im Vergleich zu Negativkontrolldaten ein Indikator für die Sensitivität des mikrobiellen Erregers gegen das Antiinfektivum sein.

Die Negativkontrolldaten können von einer Probe mit dem mikrobiellen Erreger ohne Antiinfektivum stammen. Die Daten dieser Probe können bereits vorliegen, z.B. als Daten einer Probe, die bereits zu einem früheren Zeitpunkt gemäß dem vorliegenden Verfahren analysiert wurden. Alternativ kann die Probe mit dem mikrobiellen Erreger ohne Antiinfektivum parallel zur Probe mit dem Antiinfektivum analysiert werden. In einer anderen Ausführungsform können die Negativkontrolldaten von derselben Probe, bevor diese in Kontakt mit dem Antiinfektivum gekommen ist, stammen. Außerdem könnten die Negativkontrolldaten von einer Probe eines antiinfektivaresistenten Stammes mit oder ohne Antiinfektivum stammen, insbesondere von einer Probe eines antiinfektivaresistenten Stammes.

Als Positivkontrolle könnte eine Probe eines Antiinfektivums mit einem mikrobiellen Erreger dienen, der sensitiv für dieses Antiinfektivum ist (Vergleichserreger).

Bevorzugt ist der Vergleichserreger aus der gleichen Gattung, besonders bevorzugt aus der gleichen Art des zu untersuchenden Erregers. Die Gattung und Art des unbekannten mikrobiellen Erregers können beispielsweise direkt in dem hier beschriebenen Verfahren bestimmt werden. Dann kann eine unveränderte Zellmorphologie des mikrobiellen Erregers im Vergleich zu den Positivkontrolldaten ein Indikator für eine Sensitivität des mikrobiellen Erregers gegen das Antiinfektivum sein. Darüber hinaus kann ein unverändertes Zellwachstum des mikrobiellen Erregers im Vergleich zu den Positivkontrolldaten ein Indikator für eine Sensitivität des mikrobiellen Erregers gegen das Antiinfektivum sein. Außerdem, kann ein unverändertes Raman-Spektrum des mikrobiellen Erregers im Vergleich zu den Positivkontrolldaten ein Indikator für die Sensitivität des mikrobiellen Erregers gegen das Antiinfektivum sein.

Die optische Detektion kann durch Mikroskopie, holographische Detektion oder dynamische Lichtstreuung, bevorzugt Mikroskopie erfolgen. Typischerweise ist die Mikroskopie Lichtmikroskopie. Der Begriff "Lichtmikroskopie" ist dem Fachmann bekannt und umfasst u.a. Durchlichtmikroskopie, Auflichtmikroskopie, Fluoreszenzmikroskopie, Holographie, Interferenzkontrastmikroskopie, Polarisationsmikroskopie und Konfokalmikroskopie oder Kombinationen davon. In bevorzugten Ausführungsformen wird Durchlichtmikroskopie zur optischen Detektion verwendet. In einer besonders bevorzugten Ausführungsform wird ein konfokales Mikroskop im Durchlichtmodus verwendet.

Daher eignen sich zur Ausführung des Verfahrens der Erfindung Raman-Spektrometer mit integrierten Vorrichtungen zur optischen Detektion, insbesondere Mikroskope wie Durchlichtmikroskope oder konfokale Mikroskope. Insbesondere eignen sich Raman-Spektrometer mit integriertem Durchlicht- und/oder Fluoreszenzmikroskop (z.B. alpha300 von WiTEC, Ulm, Germany, inVia confocal Raman microscope von RENISHAW, United Kingdom oder XploRA™PLUS von HORIBA). Ein derartiges Durchlicht- und/oder Fluoreszenzmikroskop ist bevorzugt ein Konfokalmikroskop. Außerdem ist es vorteilhaft, wenn eine Weißlichtquelle für die optische Detektion integriert ist. Eine Laserquelle ist insbesondere für die Raman-Spektroskopie benötigt. Das Raman-Spektrometer verfügt über einen Detektor, ein Spektrometer und üblicherweise mindestens einen Filter.

Zur Einzelzellanalyse ist ein Objektiv mit hoher numerischer Apertur, d.h. mit einer numerischen Apertur von größer oder gleich 0.8, bevorzugt größer oder gleich 0.9 verwendet.
Alternativ können einzelne Mikroorganismen durch Einsatz von inhomogenen elektrischen Feldern lokal angereichert werden (z. B. durch negative Dielektrophorese Ansammlung der Bakterien in der Mitte des Probengefäß). Anschließend können mit Hilfe von Raman-Spektroskopie Mittelwertspektren aller sich im Fokus befindenden mikrobiellen Erreger erfasst werden.

Raman-spektroskopische Verfahren zur Bestimmung von Antiinfektivaresistenzen sind dem Fachmann bekannt (Schröder et al.). Bei der Raman-Spektroskopie wird eine Probe durch monochromatisches Licht (Laser) angeregt. Dadurch entsteht an der Probe auch unelastisch gestreutes Licht, das sich in der Frequenz von der des anregenden Lichts unterscheidet. Diese Frequenzunterschiede, die sog. Raman-Verschiebung, enthalten Informationen über die Schwingungszustände der Moleküle und damit über die chemische Zusammensetzung des Mikroorganismus.

Typischerweise erfolgt die Raman-spektroskopische Bestimmung auf Einzelzellniveau. Dazu wird beispielsweise im Weißlicht eine Weitfeldaufnahme der Mikrolochkammer durchgeführt. Durch einen Bilderkennungsalgorithmus, der an die Hardware des Gerätes gekoppelt ist, werden die einzelnen Bakterien detektiert. Diese können automatisch, beispielsweise durch die Bewegung des Probentisches oder durch die Bewegung der Optik angefahren werden. Die Raman-Messung wird dann an einem einzelnen Bakterium durchgeführt. Das Signal wird in der Z-Fokusebene optimiert. Im reinen Medium kann zusätzlich der Effekt der optischen Fallen ausgenutzt werden, um einen optimalen Fokus zu erzielen. Bei optimaler Fokussierung spielt die Hintergrundfluoreszenz des Mediums kaum eine Rolle. Für eine robuste Datenanalyse können zusätzlich Wellenlängen-modulierte Anregung angewandt werden. Wenn das Signal eines einzelnen Mikroorganismus zu schwach ist, können diese auch an bestimmten Stellen im Probengefäß, z.B. über Messbereich, angereichert werden (z. B. durch Verwendung von inhomogenen, elektrischen Feldern). Die Anreicherung erfolgt dann an vorher definierten Stellen im Probengefäß, die automatisch angefahren werden können. Für die optische Analyse der Anzahl und der Morphologie werden alle im Bildfeld des Mikroskops sichtbaren Mikroorganismen erfasst und für die Auswertung verwendet. Für die Raman-spektroskopische Analyse dienen die Mikroorganismen, die durch die Wirkung des elektrischen Feldes angesammelt wurden. Die Elektrodenstruktur ist an die Größe der zu untersuchenden Mikroorganismen anzupassen.

Das Probengefäß kann ohne darauf einzuschränken, die Vertiefung (Well) einer Multiwellplatte, eine Lochkammer, ein Mikrocontainer oder der Probenbereich eines Messchips sein.

Durch die optische Detektion können die Zellzahl, die Wachstumsrate und/oder die Zellmorphologie des mikrobiellen Erregers in der Probe bestimmt werden. Dadurch lassen sich beispielsweise morphologische Veränderungen, die durch den Einfluss des Antiinfektivums hervorgerufen werden, detektieren. Durch die optionale Verwendung von inhomogenen elektrischen Feldern können außerdem Veränderungen in der Polarisierbarkeit einfacherer sichtbar gemacht werden und können so zu einer schnelleren Erkennung von Resistenzen verwandt werden. Auch die gerichtete Orientierung, die durch Anlegen eines elektrischen Feldes erreicht werden kann, kann die Streuung der ermittelten optischen Parameter reduzieren. Dadurch kann die Dauer der Bestimmung der Antiinfektivaresistenz verkürzt werden.

Der Begriff "Morphologie" bezieht sich auf Parameter, die die Form des mikrobiellen Erregers beschreiben, z.B. Größe, Rundigkeit, Flächeninhalt, Verhältnis Flächeninhalt zu Umfang, Verhältnis längste Ausdehnung (Länge) zu kürzester Ausdehnung (Breite). Bevorzugte Parameter sind Größe, Rundigkeit und Flächeninhalt. Durch automatische Bilderkennungsalgorithmen können diese Parameter oder daraus abgeleitete Kenngrößen (z. B. Parameter von Zernike-Polynomen) aus den Daten der optischen Detektion extrahiert werden.

Zur Bestimmung der quantitativen Informationen über die morphologische Änderung erfolgt die Mikroskopie auf Einzelzellniveau. Dazu wird zu jedem Zeitintervall zuerst eine Weitfeldaufnahme im Weißlicht durchgeführt und durch entsprechende Bilderkennungsalgorithmen die verschiedenen Bakterien automatisch erkannt und charakterisiert (siehe oben).

Die Bestimmung der Antiinfektivaresistenz erfolgt durch die optische Detektion und durch Raman-Spektroskopie. Das bedeutet, dass für die Auswertung die Daten der optischen Detektion, insbesondere die Mikroskopiebilddaten und die Raman-Spektren verwendet werden.

Bei der Verwendung von holographischen Ansätzen oder dynamischer Lichtstreuung können auf den Bildausschnitt gemittelte Daten erfasst werden. Insbesondere bei holographischen Ansätzen können auch durch Datenauswertung die Charakteristiken der einzelnen Mikroben bestimmt werden.

Es kann zusätzlich die Bestimmung der Wachstumsrate der mikrobiellen Erreger erfolgen. Dazu werden zu unterschiedlichen Zeitpunkten der Kultivierung der Probe Weitfeldaufnahmen getätigt. Durch automatische Bilderkennungsalgorithmen wird die Anzahl der Mikroorganismen in der Probe ermittelt. Die Wachstumsrate wird anhand der Veränderung der Anzahl der Mikroorganismen über die Zeit in der Probe ermittelt.

Die Bestimmung des mikrobiellen Erregers und ggf. dessen Antiinfektivaresistenz erfolgt durch automatisierte Analyse anhand von multivariaten statistischen Methoden, wie Hauptkomponentenanalyse, Neurale Netzwerke oder Support Vektor Maschinen und verschiedene Korrelationsalgorithmen. Diese automatisierten Analysemethoden werden mit Bilddaten und Raman-Spektren von verschiedenen Mikroorganismen (insbesondere Bakterien) mit unterschiedlichen Antiinfektiva (insbesondere Antibiotika) trainiert. Die optischen und spektralen Daten sind unabhängig, spiegeln aber die gleichen Veränderungen in den Mikroorganismen zurück. Dadurch erhöht sich durch eine Kombination der Informationen die Genauigkeit. Damit können kürzere Analysenzeiten bei hoher Genauigkeit erreicht werden. Zur kombinierten Auswertung der spektralen Informationen und der Bilddaten sind dabei zwei Ansätze möglich: 1. Aus den Bilddaten werden quantitative Parameter (z. B. Anzahl der mikrobiellen Erreger, Rundigkeit, Größe, Formfaktor (Aspektverhältnis) etc) ermittelt und diese als weitere Variable zu den Raman-Daten (Intensität über Wellenzahlen) für die multivariate, statistische Auswertung hinzugefügt; 2. Aus den Raman-Daten werden mit Hilfe statistischer Auswertung Markerbanden identifiziert (spezifisch für den Wirkmechanismus des verwendeten Antiinfektivums) und aus den relativen Intensitäten bei den Markerbanden einzelne Variablen erzeugt, die zusammen mit den quantitativen Parametern aus der optischen Analyse in einem gemischten statistischen Model ausgewertet werden können.

Typischerweise werden die Mikroorganismen (z.B. Bakterien) als reine Suspension im Verfahren der Erfindung eingesetzt. Dazu werden die Mikroorganismen (z.B. Bakterien) in die zur Analyse bestimmten Kammern einer Mikrolochplatte als Aliquote der Suspension eingebracht. Da stets das gleiche Volumen und die gleiche Konzentration für alle Kammern benötigt werden, kann die Befüllung automatisch durch entsprechende Vorrichtungen erfolgen. Durch die Gravitation sinken die Mikroorganismen (z.B. Bakterien) auf den Boden. Alternativ könnte auch eine Einbettung der Mikroorganismen in ein geeignetes Einbettungsmedium, z.B. Agarose, erfolgen. Dabei müssen entsprechende Diffusionszeiten des Antiinfektivums beachtet werden. Alternativ kann die Erregersuspension (z.B. Bakteriensuspension) durch eine geeignete Mikrofluidikplatte geleitet werden.

Der Begriffe "Mikrolochplatte" und "Mikrotiterplatte" können austauschbar verwendet werden und beziehen sich auf Titerplatten mit einem Füllvolumen von 0,1 µl bis 2000 µl, bevorzugt 0,5 µl bis 1000 µl, mehr bevorzugt 1 µl bis 500 µl, noch mehr bevorzugt 2 µl bis 200 µl, am meisten bevorzugt 5 µl bis 100 µl, zum Beispiel 10 µl. Mikrolochplatten sind üblicherweise aus Kunststoff. Bevorzugt sind Mikrolochplatten die einen Glasboden aufweisen. Der Boden kann eine dünne Beschichtung aus Agarose oder Poly-L-lysin aufweisen. Dies kann die Anreicherung der Bakterien in Glasbodennähe begünstigen. Sollen Elektroden verwendet werden (zur Erzeugung eines inhomogenen elektrischen Feldes), so werden diese ebenfalls auf die Bodenplatte aufgebracht. Eine Anordnung in den Wänden der Lochkammern ist für die hier beschriebene Anwendung auch denkbar.. In bevorzugten Ausführungsformen werden Lochkammerplatten verwendet, die das Wachstum der mikrobiellen Erreger nicht beeinflussen. Durch die Verwendung von Mikrolochplatten wird nur wenig Bakterienmaterial benötigt. Mikrolochplatten sind preiswert und damit für kostengünstige Routinediagnostikanwendungen geeignet. Der Boden der Kammer der Lochplatte sollte durch die Erreger-Suspension benetzt sein. Die Größe der Kammer kann an das Bildfeld des verwendeten Mikroskops angepasst werden.

Für die Kultivierung der Mikroorganismen sind Medien geeignet, die zur Anzucht eines breiten Spektrums von Mikroorganismen geeignet sind, beispielsweise Herz-Hirn-Bouillon (typische Zusammensetzung: Kalbshirninfusion 12,5 g/l; Rinderherzinfusion 5,0 g/l; Proteose-Pepton 10,0 g/l; Glucose 2,0 g/l; Natriumchlorid 5,0 g/l; Dinatriumhydrogenphosphat 2,5 g/l; pH 7,4 ± 0,2; z.B. von Carl Roth, Deutschland).

Die Kultivierung der mikrobiellen Erreger erfolgt bei einer Temperatur, die für die Zellteilung des Erregers möglichst optimal ist. In der Regel erfolgt die Kultivierung zwischen 25°C bis 39°C, bevorzugt zwischen 30°C und 38°C, besonders bevorzugt bei 37°C. Für die kontinuierliche Erhaltung der Temperatur kann beispielsweise eine Probenkammer eingesetzt werden, die auf die gewünschte Temperatur eingestellt ist, beispielsweise auf 37°C. Da die Raman-Spektroskopie und die optische Detektion parallel zur Kultivierung erfolgen, ist in der Vorrichtung die Probenkammer am oder um das Raman-Spektrometer mit integrierter optischer Detektionsvorrichtung so angeordnet, dass die Probe während der Raman-Spektroskopie und der optischen Detektion auf der gewünschten Temperatur gehalten werden kann. Dabei kann die Temperatursicherung sowohl lokal (z. B. durch Heizfolie) oder global (umfaßt Mikrolochplatte und Teile des optischen Aufbaus) erfolgen. Die Lichtleitung kann dabei durch optische Fasern erfolgen.

In einer besonderen Ausführungsform besitzt die Vorrichtung zwei unterschiedlich temperierte Probenkammern. Beispielsweise kann eine Probenkammer auf 30°C und die zweite Probenkammer auf 37°C eingestellt sein. Die Probe wird zunächst in eine der beiden Kammern, beispielsweise in die 37°C temperierte Kammer eingeführt und die mikrobiellen Erreger durch optische Detektion und/oder Raman-Spektroskopie identifiziert. Wenn von dem identifizierten Erreger bekannt ist, dass dieser besser bei 30°C wächst, wird die Mikrolochplatte in die zweite Kammer eingeführt. Die Einführung der Probe in die Kammer kann automatisch erfolgen. Alternativ könnte eine Probenkammer verwendet werden und die Temperatur der Probenkammer nach der Identifizierung des mikrobiellen Erregers an die optimale Wachstumstemperatur des Erregers angepasst werden. In einer weiteren Ausführungsform, werden parallel zwei Proben, die bezüglich dem mikrobiellen Erregers und (ggf.) dem Antibiotikum gleich sind, parallel in zwei unterschiedlich temperierten Kammern, z.B. in einer auf 37°C und einer auf 30°C temperierten Kammer, kultiviert.

Das beschriebene Verfahren ist prinzipiell für alle Antiinfektiva (insbesondere Antibiotika) geeignet. Typischerweise wird als Kontrolle eine reine Probe ohne Antiinfektiva-Zusatz oder eine reine Probe ohne Antiinfektivaresistenz verwendet. Alternativ können Vergleichsdatensätze verwendet werden, die für den entsprechenden Erreger (z.B. Vergleichserreger der gleichen Art) in Kombination mit den entsprechenden Antiinfektiva bereits vorhanden sind. In einer Ausführungsform werden Vergleichsdatensätze verwendet, bei denen das gleiche Antiinfektivum wie der zu untersuchenden Probe zugesetzt ist, sich aber bezüglich des mikrobiellen Erregers von der zu untersuchenden Probe unterscheiden. Das Antiinfektivum kann bereits lyophilisiert in den Kammern der Lochplatte vorliegen. Je nach Herkunft des Probenmaterials (Blutkultur, Atemwege, Harnwege etc.) kann eine entsprechende Kartusche ausgewählt und befüllt werden.

Das Antiinfektivum wird nach der Raman-spektroskopischen Identifizierung der Keime in die Kammern dosiert. Dazu können beispielsweise Vorratsgefäße mit Injektionsanschlüssen in das Gerät eingebaut werden. Es wird empfohlen bei dieser Version einen Teststamm mit bekannter Empfindlichkeit in einer Kammerreihe mitlaufen zu lassen, um gleichzeitig eine Kontrolle über die Wirksamkeit des Antiinfektivums zu haben.

In einer spezifischen Ausführungsform wird eine große Menge der Bakteriensuspension (ausreichend für die benötigten Antibiotikatestungen plus Kontrolle) in eine zentrale Kammer eingefüllt. Nach Raman-spektroskopischer Identifizierung der Erreger im Gerät wird eine Mikrolochplatte ausgewählt, die die relevanten Antibiotika in den relevanten Konzentrationen enthält, und die Bakteriensuspension mikrofluidisch in die entsprechenden Kammern geleitet.

In einer Ausführungsform, wird das Verfahren mit mindestens einer Kontrollprobe durchgeführt. Diese Kontrollprobe kann den mindestens einen mikrobiellen Erreger ohne Antiinfektivum enthalten. Dadurch kann die Wachstumsrate und die Zellmorphologie der Probe(n) umfassend den mikrobiellen Erreger und ein Antiinfektivum mit der Wachstumsrate und der Zellmorphologie der Kontrollprobe(n) umfassend den mikrobiellen Erreger ohne Antiinfektivum verglichen werden.
Zusätzlich kann das Verfahren in einer Probe, die einen antiinfektivasensitiven Teststamm umfasst, durchgeführt werden.

In einer Ausführungsform wird das Verfahren an mehreren Proben, die jeweils einen mikrobiellen Erreger und jeweils ein anderes Antiinfektivum enthalten durchgeführt. Da das Verfahren der vorliegenden Erfindung leicht parallelisiert werden kann, ist es somit möglich gleichzeitig die Resistenz des mikrobiellen Erregers gegen mehrere Antiinfektiva zu testen. Dies ist aufgrund der immer häufiger auftretenden multiresistenten mikrobiellen Erreger, insbesondere von multiresistenten Bakterien (z.B. Methicillin-resistenter *Staphylococcus aureus* (MRSA), multiresistnte *Clostridium difficile* aber auch multiresistente Gram-negative Bakterien (MRGN) wie z. B. Multiresistenter *Pseudomonas aeruginosa, Acinetobacter baumannii,* oder Enterobakterien wie *Escherichia coli* und *Klebsiella pneumoniae*) von enormer Wichtigkeit.

In einer Ausführungsform wird das Verfahren an mehreren Proben, die jeweils einen mikrobiellen Erreger und ein Antiinfektivum in jeweils unterschiedlichen Konzentrationen enthalten, durchgeführt. Damit kann auf einfache Weise die minimale Hemmkonzentration bestimmt werden.

Es ist offensichtlich, dass sich die vorliegende Erfindung ebenso so zur parallelen Analyse von unterschiedlichen Antiinfektiva jeweils in unterschiedlichen Konzentrationen eignet.

In spezifischen Ausführungsformen findet die optische Detektion zu Beginn der Kultivierung und in Zeitintervallen von höchstens 120 Minuten, bevorzugt 60 Minuten, besonders bevorzugt höchstens 30 Minuten, mehr bevorzugt höchstens 15 Minuten, noch mehr bevorzugt höchstens 5 Minuten statt. Die Raman-Spektroskopie findet zu Beginn der Kultivierung und in Zeitintervallen von höchsten 120 Minuten, bevorzugt höchstens 60 Minuten, besonders bevorzugt höchstens 30 Minuten statt.

Mit Hilfe des Verfahrens der Erfindung kann die Bestimmung des mikrobiellen Erregers und dessen Antiinfektivaresistenz innerhalb von 180 Minuten, bevorzugt 150 Minuten, mehr bevorzugt 120 Minuten, besonders bevorzugt 60 Minuten abgeschlossen werden. Als verwendete Technologien kommen zur Bestimmung der Gattung und Art des mikrobiellen Erregers die Raman-Spektroskopie und zur Bestimmung der Antiinfektivaempfindlichkeit ein kombiniertes Verfahren aus Raman-Spektroskopie und optische Analyse zum Einsatz. Identifizierung und Resistenzbestimmung können dabei in der gleichen Vorrichtung erfolgen. Es sind keine weiteren Probenvorbehandlungsschritte notwendig. Das benötigte Probenmaterial ist minimal.

Typischerweise bezieht sich das Verfahren auf die Identifizierung von pathogenen mikrobiellen Erregern. Daher wird die Probe aus einem Individuum, bevorzugt einem Säugetier, besonders bevorzugt einem Menschen entnommen. Die Probe kann eine Körperflüssigkeit, z.B. eine Urinprobe, oder eine in ein Medium überführte Mikrokolonie sei. Die Urinprobe wird typischerweise filtriert, mit Hilfe eines Filters, der Verunreinigungen absondert, jedoch mikrobielle Erreger passieren lässt. Komplexe Proben, die üblicherweise viele Bestandteile, wie beispielsweise Blutproben, oder auch Besiedlungsflora aufweisen, wie beispielsweise, aspiratorisches Material, werden, wie in den mikrobiologischen Laboren etabliert und für den Fachmann bekannt, ausgestrichen und für kurze Zeit, z.B. weniger als 6 h, bevorzugt weniger als 4 h, mehr bevorzugt weniger als 3 h, besonders bevorzugt weniger aus 2 h kultiviert. Da nur wenig Bakterienmaterial benötigt wird, sind kurze Inkubationszeiten von ca. 2-3 h ausreichend, um genug Probenmaterial zu erhalten. Alternativ sind auch mikrofluidische Aufreinigungsschritte denkbar, die die mikrobiellen Erreger direkt aus dem komplexen Patientenmaterial für die weitere Analyse mit der hier beschriebenen Erfindung zugänglich machen. Einer solche Voraufreinigung kann auch an das hier beschriebene Titerplattensystem direkt gekoppelt werden.

Für die Verfahren gemäß der Erfindung sind lediglich 50 bis 1000, bevorzugt 100 bis 1000 mikrobielle Erreger in einer Probe notwendig. Typischerweise enthält eine Probe 5 x 10¹ bis 1 x 10⁵ bevorzugt 1 x 10² bis 1 x 10⁵, mehr bevorzugt 5 x 10² bis 1 x 10⁵ mikrobielle Erreger. In besonders bevorzugten Ausführungsformen enthält die Probe 1 x 10³ bis 1 x 10⁴ mikrobielle Erreger. Da nur eine geringe Anzahl von Erregern sowohl zur Bestimmung der Erreger als auch zur Bestimmung derer Antiinfektivaresistenz notwendig ist, kann auf einen oder mehrere Vorkulturschritte, die bei den Verfahren im Stand der Technik nötig sind, verzichtet werden. Da diese Vorkulturschritte üblicherweise zwischen 6 und 24 Stunden dauern, kann im vorliegenden Verfahren das Ergebnis ab Probennahme wesentlich schneller erzielt werden. Die Dauer des Verfahrens der Erfindung zur Bestimmung der Erreger und/oder deren Antiinfektivaresistenzen kann entsprechend verkürzt werden. Somit ist eine Bestimmung des mikrobiellen Erregers und dessen Antiinfektivaresistenz innerhalb von 180 Minuten, bevorzugt 150 Minuten, besonders bevorzugt 120 Minuten nach Probenentnahme möglich.

Eine spezifische Ausführungsform betrifft ein Verfahren zur Bestimmung des mikrobiellen Erregers und dessen Antiinfektivaresistenzen, umfassend die Schritte:
- Kultivierung einer Probe umfassend mindestens einen mikrobiellen Erreger und ein Antiinfektivum;
- Kultivierung einer Kontrollprobe umfassend mindestens einen mikrobiellen Erreger ohne Antiinfektivum;
- Raman-Spektroskopie der Proben zu mindestens zwei Zeitpunkten während der Kultivierung der Probe;
- optische Detektion der Proben zu mindestens zwei Zeitpunkten während der Kultivierung der Probe;

Ein weiterer Aspekt betrifft ein System zur Bestimmung eines mikrobiellen Erregers umfassend:
- Raman-Spektrometer zur Bereitstellung von spektroskopischen Daten des mikrobiellen Erregers,
- Vorrichtung zur optischen Detektion des mikrobiellen Erregers zur Bereitstellung von optischen Daten des mikrobiellen Erregers,
wobei das System zur Bestimmung der Morphologie des mikrobiellen Erregers durch Analyse der optischen Daten und zur Bestimmung des mikrobiellen Erregers basierend auf einer Kombination dem Raman-Spektrum und der morphologischen Daten des mikrobiellen Erregers ausgeführt ist.

Eine Ausführungsform bezieht sich auf ein System zur Bestimmung eines mikrobiellen Erregers umfassend:
- Raman-Spektrometer zur Bereitstellung von spektroskopischen Daten des mikrobiellen Erregers,
- Vorrichtung zur optischen Detektion des mikrobiellen Erregers zur Bereitstellung von optischen Daten des mikrobiellen Erregers,
wobei das System zur Bearbeitung eines Raman-Spektrums des mikrobiellen Erregers, zur Bestimmung der Morphologie des mikrobiellen Erregers durch Analyse der optischen Daten und zur Bestimmung des mikrobiellen Erregers basierend auf einer Kombination des Raman-Spektrums und der morphologischen Daten des mikrobiellen Erregers ausgeführt ist.

In einer spezifischen Ausführungsform, ist das System ferner zur Bestimmung der Antiinfektivaresistenz basierend auf einer Kombination des bestimmten Raman-Spektrums und der bestimmten Morphologie des mikrobiellen Erregers ausgeführt.

Ein weiterer Aspekt bezieht sich auf ein Programmelement zur Bestimmung eines Erregers, das, wenn es auf einem Prozessor ausgeführt wird, den Prozessor anleitet, die folgenden Schritte durchzuführen:
- Bestimmen einer Morphologie des mikrobiellen Erregers basierend auf den Daten einer Vorrichtung zur optischen Detektion des mikrobiellen Erregers , und
- Bestimmen des mikrobiellen Erregers basierend auf einem Raman-Spektrum und der Morphologie des mikrobiellen Erregers.

Eine andere Ausführungsform bezieht sich auf ein Programmelement zur Bestimmung eines Erregers, das, wenn es auf einem Prozessor ausgeführt wird, den Prozessor anleitet, die folgenden Schritte durchzuführen:
- Bearbeitung eines Raman-Spektrums des mikrobiellen Erregers,
- Bestimmen einer Morphologie des mikrobiellen Erregers basierend auf den Daten einer Vorrichtung zur optischen Detektion des mikrobiellen Erregers, und
- Bestimmen des mikrobiellen Erregers basierend auf dem bearbeiteten Raman-Spektrum und der Morphologie des mikrobiellen Erregers.

Der Begriff "Bearbeitung des Raman-Spektrums" umfasst gängige dem Fachmann bekannte Methoden zur Signaloptimierung des Raman-Spektrums, wie beispielsweise Untergrundkorrektur, Normalisierung oder Wellenzahlkorrektur.

In einer Ausführungsform leitet das Programmelement den Prozessor an, ferner den folgenden Schritt auszuführen:
- Bestimmen der Antiinfektivaresistenz des mikrobiellen Erregers basierend auf dem Raman-Spektrum und der Morphologie des mikrobiellen Erregers nach Interaktion mit dem Antiinfektivum.

Das Programmelement kann ein Teil eines Computerprogrammes sein, aber es kann auch eigenständig sein. Beispielsweise kann das Programmelement zum Aktualisieren eines bereits existierenden Programmelements verwendet werden.

Ein weiterer Aspekt bezieht sich auf ein computerlesbares Medium, auf dem ein Programmelement gespeichert ist, das, wenn es auf einem Prozessor ausgeführt wird, den Prozessor anleitet, die folgenden Schritte durchzuführen:
- Bestimmen einer Morphologie des mikrobiellen Erregers basierend auf den Daten einer Vorrichtung zur optischen Detektion des Erregers, und
- Bestimmen des mikrobiellen Erregers basierend auf dem Raman-Spektrum und der Morphologie des mikrobiellen Erregers.

Eine weitere Ausführungsform bezieht sich auf ein computerlesbares Medium, auf dem ein Programmelement gespeichert ist, das, wenn es auf einem Prozessor ausgeführt wird, den Prozessor anleitet, die folgenden Schritte durchzuführen:
- Bearbeitung eines Raman-Spektrums des mikrobiellen Erregers,
- Bestimmen einer Morphologie des mikrobiellen Erregers basierend auf den Daten einer Vorrichtung zur optischen Detektion des Erregers, und
- Bestimmen des mikrobiellen Erregers basierend auf dem bearbeiteten Raman-Spektrum und der Morphologie des mikrobiellen Erregers.

In einer Ausführungsform kann das computerlesbares Medium den Prozessor anleiten ferner den folgenden Schritt durchzuführen: Bestimmen der Antiinfektivaresistenz des mikrobiellen Erregers basierend auf dem Raman-Spektrum und der Morphologie des mikrobiellen Erregers.

Das computerlesbare Medium kann als Speichermedium, z.B. als USB-Stick, CD oder DVD, Datenspeichergerät, Festplatte oder jedes beliebige andere Medium, auf dem ein wie oben beschriebenes Programmelement gespeichert werden kann, betrachtet werden.

### Ausführungsbeispiel 1

- Erstellung einer Bakteriensuspension und Einbringung dieser in Kammern einer Mikrolochkammerplatte (Füllvolumen der Kammer ≤100 µl, Aliquot 10 µl): Eine Mikrolochkammer enthält kein Antibiotikum, die anderen Mikrolochkammern enthalten eines bzw. jeweils verschiedene Antibiotika.
- Raman-spektroskopische Identifizierung der Erreger durch Aufnahme von Einzelspektren von mehreren einzelnen Erregern in der Messkammer und Abgleich der Spektren mit einer Datenbank zur Bestimmung der Bakterienart: Durch entsprechende Bilderkennungsalgorithmen, die an die Hardware des Gerätes gekoppelt sind, können verschiedene Bakterien automatisch angefahren werden. Inkubation der Bakterien bei 37°C.
- Verfolgen des Bakterienwachstums mit Hilfe von Weißlichtmikroskopie und anschließender Bildauswertung: Dazu wird alle ca. 15 min. eine Weitfeldaufnahme jeder Lochkammer gemacht. Durch Bilderkennungsalgorithmen werden quantitative Informationen über die morphologischen Änderungen gewonnen. In der Auswertung werden diese Informationen relativ zur Kontrolle ohne Antibiotikum ausgewertet.
- Raman-spektroskopische Charakterisierung: Nach 60 und nach 120 Minuten wird eine Raman-spektroskopische Charakterisierung anhand von ca. 10-20 Bakterien in den einzelnen Kammern durchgeführt. Durch die Analyse der Raman-Spektren werden quantitative Informationen über den biochemischen Fingerabdruck, d.h. Informationen zur biochemischen Zusammensetzung der Bakterien, gewonnen. In der Auswertung werden diese Informationen relativ zur Kontrolle ohne Antibiotikum bestimmt. Die Bakterien werden dabei, basierend auf den Bildinformationen, durch automatisiertes Verfahren des Probentisches automatisch angefahren.
- Automatisierte Analyse der Antibiotikaresistenz durch Integration der Daten bezüglich der Wachstumskurven, der quantitativen Informationen über morphologische Änderungen sowie der Raman-spektroskopischen Charakterisierung: Dazu werden die spektralen Veränderungen der Bakterien-Raman-Spektren in den Kammern mit Antibiotikumzusatz mit denen in der Kontrollkammer ohne Antibiotikumzusatz verglichen. Treten die für den Wirkmechanismus des Antibiotikums charakteristischen Veränderungen auf, so ist dies ein Indikator für Sensitivität. Sind keine Unterschiede feststellbar bzw. die für einen bestimmten Resistenzmechanismus charakteristischen Unterschiede, so ist dies ein Indikator für Resistenz. Der Vergleich erfolgt dabei durch statistische Auswertung der (vorbehandelten) Raman-Spektren unter Verwendung multivariater Methoden (z. B. Hauptkomponentenanalyse, Liniare Diskriminanzanalyse, Support-Vektor-Analyse oder andere). Analog werden aus der Analyse der Wachstumscharakteristik und der morphologischen Änderungen Indikatoren für Resistenz und Sensitivität erhalten. Diese drei Indikatoren werden mit Hilfe eines gewichteten statistischen Verfahrens dazu verwandt, Aussagen über die Resistenz bzw. Sensitivität des untersuchten Bakteriums gegenüber den verschiedenen Antibiotika zu treffen.

Die optische Detektion erfolgt an einem Raman-Spektrometer mit integriertem Konfokalmikroskop mit Weißlichtbeleuchtung, (z.B. inVia confocal Raman microscope von RENISHAW, United Kingdom oder XploRA™PLUS von HORIBA).

### Ausführungsbeispiel 2

- Einbringung von Aliquots einer Bakteriensuspension in Kammern einer Mikrolochkammerplatte (Füllvolumen der Kammer ≤100 µl, Aliquot 10 µl). In jeder Kammer befindet sich eine andere Konzentration eines Antibiotikums, dessen Resistenz getestet werden soll (Konzentrationsbereich: 0 µg/ml bis 4-fache Breakpoint nach EUCAST). Die Kammern sind mit Elektroden ausgestattet, die die Erzeugung eines elektrischen Feldes ermöglichen.
- Anschalten des elektrischen Feldes. Die Bakterien werden an einer definierten Stelle im Lochkammer konzentriert. Die Raman-spektroskopische Identifizierung der Erreger erfolgt durch Aufnahme von Raman-Spektren an der definierten Stelle im Messchip und Abgleich der Spektren mit einer Datenbank zur Bestimmung der Bakterienart. Die folgenden Inkubation der Bakterien bei 37°C kann ohne die Erzeugung eines elektrischen Feldes stattfinden.
- Verfolgen des Bakterienwachstums mit Hilfe von Weißlichtmikroskopie und anschließender Bildauswertung: Dazu wird alle ca. 5- 15 min. eine Weitfeldaufnahme in jeder Lochkammer gemacht. Durch Bilderkennungsalgorithmen werden quantitative Informationen über die morphologischen Änderungen gewonnen. Zur Anreicherung, und zur Ausrichtung der Bakterien (insbesondere für stäbchenförmige Bakterien interessant) kann das elektrische Feld unmittelbar vor Aufnahme des Mikroskopbildes eingeschaltet werden. Die Auswertung umfasst die dynamische Änderung der optischen Parameter (und ggf. der Polarisierbarkeit). Als Kontrolle dienen dabei sowohl Zeitpunkt 0 min und das Aliquot ohne Antibiotikum.
- Raman-spektroskopische Charakterisierung: Nach 30, 60 und nach 90 Minuten erfolgt eine Raman-spektroskopische Charakterisierung innerhalb der durch das elektrische Feld angereicherten Bakterienwolke. Dabei werden mehrere Spektren in jeder Messkammer aufgenommen. Durch die Analyse der Raman-Spektren werden quantitative Informationen über den biochemischen Fingerabdruck, d.h. Informationen zur biochemischen Zusammensetzung der Bakterien, gewonnen. Die Auswertung kann je nach Antibiotikum die Bildung von Bandenverhältnissen umfassen oder multivariate statistische Verfahren im Vergleich zur Kontrolle ohne Antibiotikum.
- Automatisierte Analyse der Antibiotikaresistenz und Bestimmung der Mindesthemmkonzentration: Dazu werden sowohl Bildinformationen als auch Raman-Daten zusammen für jede Antibiotika-Konzentration ausgewertet. Dies umfasst Informationen zu bakteriellem Wachstum, quantitative Parameter der Morphologie als auch charakteristische Raman-Banden. Für jede Antibiotika-Konzentration wird festgestellt, ob die zugegebene Antibiotika-Konzentration ausreichend war, um das Bakterienwachstum zu hemmen oder nicht. Die Mindesthemmkonzentration ergibt sich als die Konzentration, bei der ein Schwellenwert (z. B. Bandenverhältnis in den Raman-Spektren oder bestimmtes Ausmaß der Morphologieveränderung oder bestimmte Reduktion des Bakterienwachstums im Vergleich zur unbehandelten Probe) gerade nicht erreicht bzw. noch nicht überschritten wird. Die Schwellenwerte sind für bestimmte Antibiotikawirkklassen und Einwirkdauern charakteristisch. Durch Vergleich der so bestimmten Mindesthemmkonzentration mit den Grenzwerten der Empfindlichkeit (z. B. nach EUCAST-Richtlinie) erfolgt die Einstufung der unbekannten, zu charakterisierenden Bakterien in die Resistenzlevel sensibel, intermediär (für einige Bakterien-Antibiotika-Kombinationen definiert) oder resistent.

Nachfolgend wird anhand der beigefügten Zeichnung näher auf ein Ausführungsbeispiel der Erfindung eingegangen:

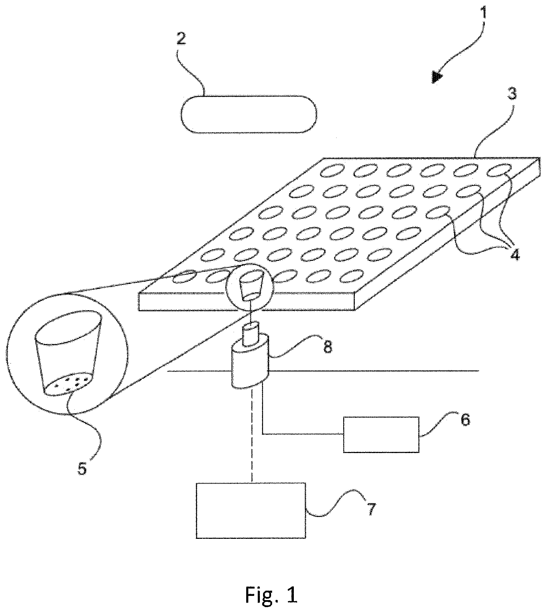

Fig. 1: Übersicht über ein System zur Bestimmung eines mikrobiellen Erregers
   In Fig. 1 ist ein System zur Bestimmung eines mikrobiellen Erregers (1) gezeigt, das eine Kamera zur optischen Detektion aufweist. Die zu bestimmenden Erreger (5) befinden sich in einer Lochkammer einer Lochkammerplatte (3), die mehrere Lochkammem (4) aufweist. Die optisch-morphologische Detektion erfolgt über eine Kamera (2). Zur Beleuchtung dient dabei eine Weisslichtquelle (9), die durch das Objektiv (8) die Probe beleuchtet. Laser (6) und dispersives Element mit Detektor (7) bildend das Raman-Spektrometer. Der Anregungslichtweg, sowie der Detektionslichtweg passieren das Objektiv (8). Der Detektor (7) dient der Raman-spektroskopischen Detektion. Die Recheneinheit (10) dient der Bestimmung der Erreger und ggf. deren Antiinfektivaresistenz durch Bestimmen einer Morphologie der Erreger basierend auf den Daten der Kamera und Bestimmen der Erreger (und ggf. deren Antiinfektivaresistenz) basierend auf dem Raman-Spektrum und der Morphologie des mikrobiellen Erregers.

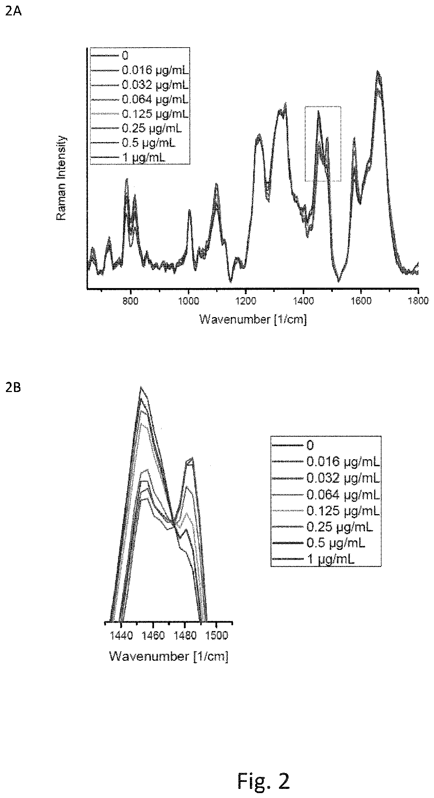

Fig. 2: 2A. Raman-Spektren eines E. coli-Stammes nach 90 Minuten Einwirkzeit von unterschiedlichen von Konzentrationen Ciprofloxacin. In Übereinstimmung mit klassischen mikrobiologischen Methoden wurde die MHK dieses Stammes zu 0.032 µg Ciprofloxacin/ml bestimmt. Damit ist dieser Stamm Ciprofloxacin-empfindlich. 2B. Vergrößerung eines Ausschnittes aus Fig. 2A (die Reihenfolge der Spektren folgt für die Bande bei ∼1485 cm⁻¹ den auf der rechten Seite angegebenen Konzentrationen, für die Raman-Bande um 1450 cm⁻¹ ist die Reihenfolge genau umgekehrt).

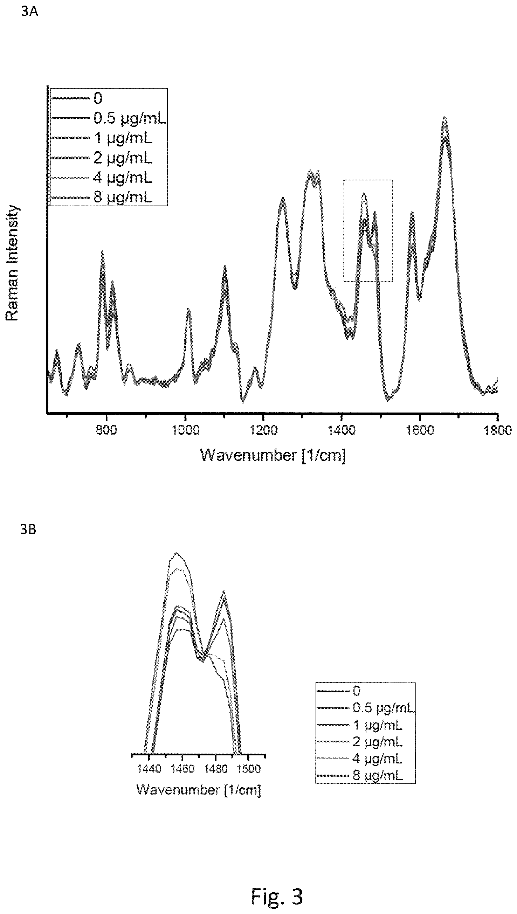

Fig. 3: 3A. Raman-Spektren eines anderen *E*. *coli*-Stammes nach 90 Minuten Einwirkzeit von unterschiedlichen Konzentrationen Ciprofloxacin. In Übereinstimmung mit klassischen mikrobiologischen Methoden wurde die MHK dieses Stammes zu 1 µg Ciprofloxacin/ml bestimmt. Damit ist dieser Stamm Ciprofloxacin-resistent. 3B. Vergrößerung eines Ausschnittes von Fig. 3A (die Reihenfolge der Spektren folgt für die Bande bei ∼1485 cm⁻¹ den auf der rechten Seite angegebenen Konzentrationen, für die Raman-Bande um 1450 cm⁻¹ ist die Reihenfolge genau umgekehrt).

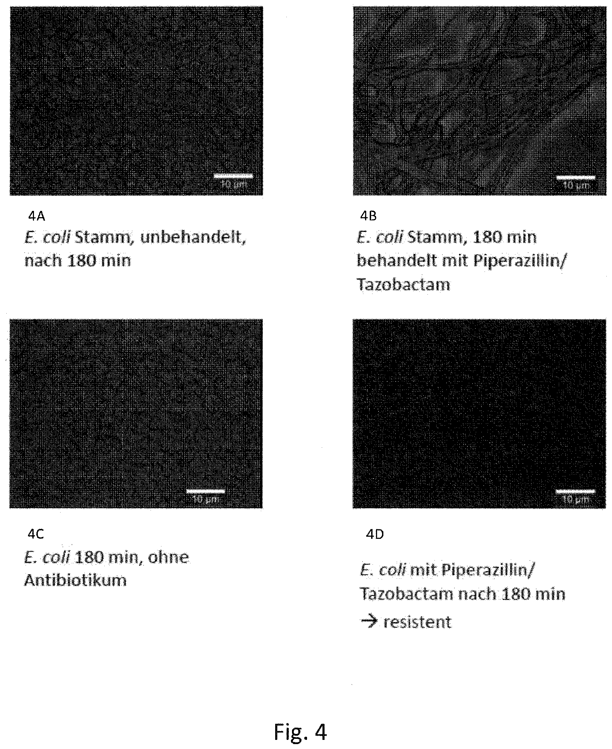

Fig 4: Morphologie von *E.coli* Stämmen ohne Antibiotikabehandlung (4A und 4C) und 180 min nach Behandlung mit Piperazillin/Tazobactam (4B: sensitiver Stamm; 4D: resistenter Stamm).

## Patentansprüche

1. Verfahren zur Bestimmung eines mikrobiellen Erregers und dessen Antiinfektivaresistenz in einer Probe umfassend den mikrobiellen Erreger und ein Antiinfektivum, umfassend die Schritte:
- Kultivierung der Probe umfassend mindestens einen mikrobiellen Erreger und ein Antiinfektivum;
- Bestimmen von Raman-Spektren des mikrobiellen Erregers durch Raman-Spektroskopie der Probe unter mindestens zwei unterschiedlichen Antiinfektivum-Bedingungen,
- Bestimmen von quantitativen Morphologieparametern des mikrobiellen Erregers durch optische Detektion der Probe unter mindestens zwei unterschiedlichen Antiinfektivum-Bedingungen,
- Bestimmen des mikrobiellen Erregers basierend auf den Raman-Spektren und den quantitativen Morphologieparametern des mikrobiellen Erregers,
- Bestimmen der Antiinfektivaresistenz des mikrobiellen Erregers basierend auf den Raman-Spektren und den quantitativen Morphologieparametern des mikrobiellen Erregers.

2. Verfahren nach Anspruch 1, wobei die mindestens zwei unterschiedlichen Antiinfektivum-Bedingungen zwei unterschiedliche Antiinfektivum-Konzentrationen und/oder zwei unterschiedliche Wirkdauern des Antiinfektivums sind.

3. Verfahren gemäß einem der Ansprüche 1 oder 2, umfassend die Schritte:
- Bestimmen der Raman-Spektren des mikrobiellen Erregers durch Raman-Spektroskopie der Probe zu mindestens zwei Zeitpunkten während der Kultivierung der Probe,
- Bestimmen der quantitativen Morphologieparameter des mikrobiellen Erregers durch optische Detektion der Probe zu mindestens zwei Zeitpunkten während der Kultivierung der Probe,
- Bestimmen des mikrobiellen Erregers basierend auf den Raman-Spektren und den quantitativen Morphologieparametern des mikrobiellen Erregers,
- Bestimmen der Antiinfektivaresistenz des mikrobiellen Erregers basierend auf den Raman-Spektren und den quantitativen Morphologieparametern des mikrobiellen Erregers.

4. Verfahren nach einem der vorhergehenden Ansprüche, wobei die optische Detektion durch Mikroskopie, holographische Detektion oder dynamische Lichtstreuung ist, wobei die Mikroskopie Durchlichtmikroskopie oder Fluoreszenzmikroskopie ist.

5. Verfahren nach einem der vorhergehenden Ansprüche, ferner umfassend den Schritt:
- Bestimmen des Zellwachstums des mikrobiellen Erregers durch optische Detektion;
wobei die Bestimmung der Antiinfektivaresistenz auf den bestimmten Raman-Spektren, der bestimmten quantitativen Morphologieparametern und dem bestimmten Zellwachstum des mikrobiellen Erregers basiert.

6. Verfahren nach einem der vorhergehenden Ansprüche, wobei der mindestens eine mikrobielle Erreger ein Bakterium und das Antiinfektivum ein Antibiotikum ist.

7. Verfahren nach einem der vorhergehenden Ansprüche, wobei bei dem Bestimmen der Antiinfektivaresistenz des mikrobiellen Erregers ein erster Indikator für die Resistenz des mikrobiellen Erregers gegen das Antiinfektivum verwendet wird, wobei der erste Indikator für die Resistenz des mikrobiellen Erregers gegen das Antiinfektivum eine unveränderte Zellmorphologie des mikrobiellen Erregers im Vergleich zu Negativkontrolldaten ist.

8. Verfahren nach Anspruch 7, wobei bei dem Bestimmen der Antiinfektivaresistenz des mikrobiellen Erregers ein zweiter Indikator für die Resistenz des mikrobiellen Erregers gegen das Antiinfektivum verwendet wird, wobei der zweite Indikator für die Resistenz des mikrobiellen Erregers gegen das Antiinfektivum eine unverändertes oder eine für einen bestimmten Resistenzmechanismus charakteristisch verändertes Raman-Spektrum des mikrobiellen Erregers im Vergleich zu Negativkontrolldaten ist und/oder wobei bei dem Bestimmen der Antiinfektivaresistenz des mikrobiellen Erregers ein dritter Indikator für die Resistenz des mikrobiellen Erregers gegen das Antiinfektivum verwendet wird, wobei der dritte Indikator für die Resistenz des mikrobiellen Erregers gegen das Antiinfektivum ein unverändertes Zellwachstum des mikrobiellen Erregers im Vergleich zu Negativkontrolldaten ist.

9. Verfahren nach Anspruch 7 oder 8, wobei die Negativkontrolldaten von einer Probe stammen, die den mikrobiellen Erreger ohne Antiinfektivum umfasst.

10. Verfahren nach einem der vorhergehenden Ansprüche, wobei das Verfahren zusätzlich mit einer Kontrollprobe enthaltend den mindestens einen mikrobiellen Erreger ohne Antiinfektivum durchgeführt wird und/oder wobei das Verfahren weniger als 180 Minuten, bevorzugt weniger als 150 Minuten, noch mehr bevorzug weniger als 120 Minuten, am meisten bevorzugt weniger als 60 Minuten dauert.

11. Verfahren nach einem der vorhergehenden Ansprüche, wobei die Probe von einem Individuum, bevorzugt einem Säugetier, besonders bevorzugt einem Menschen entnommen ist und/oder 1*10² bis 1*10⁵, bevorzugt 5*10³ bis 1*10⁴ mikrobielle Erreger enthält.

12. Verfahren nach einem der vorhergehenden Ansprüche, wobei in der Probe ein elektrisches inhomogenes Feld erzeugt wird.

## Claims

1. A method for determining a microbial pathogen and an anti-infective resistance thereof in a sample comprising the microbial pathogen and an anti-infective, comprising the following steps:
- cultivating the sample comprising at least one microbial pathogen and an anti-infective;
- determining Raman spectra of the microbial pathogen by Raman spectroscopy of the sample under at least two different anti-infective conditions,
- determining quantitative morphology parameters of the microbial pathogen by optical detection of the sample under at least two different anti-infective conditions,
- determining the microbial pathogen based on the Raman spectra and the quantitative morphology parameters of the microbial pathogen,
- determining the anti-infective resistance of the microbial pathogen based on the Raman spectra and the quantitative morphology parameters of the microbial pathogen.

2. The method according to claim 1, wherein the at least two different anti-infective conditions are two different concentrations of the anti-infective and/or two different durations of action of the anti-infective.

3. The method according to any of the claims 1 or 2, comprising the following steps:
- determining the Raman spectra of the microbial pathogen by Raman spectroscopy of the sample at at least two time-points during cultivation of the sample,
- determining the quantitative morphology parameters of the microbial pathogen by optical detection of the sample at at least two time-points during cultivation of the sample,
- determining the microbial pathogen based on the Raman spectra and the quantitative morphology parameters of the microbial pathogen,
- determining the anti-infective resistance of the microbial pathogen based on the Raman spectra and the quantitative morphology parameters of the microbial pathogen.

4. The method according to any one of the preceding claims, wherein the optical detection is by microscopy, holographic detection or dynamic light scattering, wherein the microscopy is transmitted light microscopy or fluorescence microscopy.

5. The method according to any one of the preceding claims, further comprising the step:
- determining the cell growth of the microbial pathogen by optical detection,
wherein the determination of the anti-infective resistance is based on the determined Raman spectra, the determined quantitative morphology parameters and the determined cell growth of the microbial pathogen.

6. The method according to any one of the preceding claims, wherein the at least one microbial pathogen is a bacterium and the anti-infective is an antibiotic.

7. The method according to any one of the preceding claims, wherein, when determining the anti-infective resistance of the microbial pathogen, a first indicator for the resistance of the microbial pathogen to the anti-infective is used,
wherein the first indicator for the resistance of the microbial pathogen to the anti-infective is an unchanged cell morphology of the microbial pathogen in comparison to negative control data.

8. The method according to claim 7, wherein, when determining the anti-infective resistance of the microbial pathogen, a second indicator for the resistance of the microbial pathogen to the anti-infective is used,
wherein the second indicator for the resistance of the microbial pathogen to the anti-infective is a Raman spectrum of the microbial pathogen that is unchanged or that is changed in a way that is characteristic for a specific resistance mechanism in comparison to negative control data and/or
wherein, when determining the anti-infective resistance of the microbial pathogen, a third indicator for the resistance of the microbial pathogen to the anti-infective is used,
wherein the third indicator for the resistance of the microbial pathogen to the anti-infective is an unchanged cell growth of the microbial pathogen in comparison to negative control data.

9. The method according to any one of claims 7 or 8, wherein the negative control data is from a sample comprising the microbial pathogen without anti-infective.

10. Method according to any one of the preceding claims, wherein the method is additionally performed with a control sample containing the at least one microbial pathogen without anti-infective and/or wherein the method takes less than 180 minutes, preferably less than 150 minutes, more preferably less than 120 minutes, most preferably less than 60 minutes.

11. The method according to any one of the preceding claims, wherein the sample is derived from a subject, preferably from a mammal, more preferably from a human and/or contains 1*10² to 1*10⁵, preferably 5*10³ to 1*10⁴ microbial pathogens.

12. The method according to any one of the preceding claims, wherein an inhomogeneous electric field is produced in the sample.

## Revendications

1. Procédé visant à identifier un agent microbien et à déterminer sa résistance aux agents anti-infectieux au sein d'un échantillon qui comprend le agent microbien et un agent anti-infectieux, comprenant les étapes consistant à :
- mettre en culture ledit échantillon qui comprend au moins un agent microbien et un agent anti-infectieux;
- déterminer des spectres Raman dudit agent microbien en soumettant ledit échantillon à une spectroscopie Raman réalisée dans au moins deux conditions différentes en termes d'agent anti-infectieux,
- déterminer des paramètres quantitatifs de morphologie dudit agent microbien en soumettant ledit échantillon à une détection optique réalisée dans au moins deux conditions différentes en termes d'agent anti-infectieux,
- identifier l'agent microbien à partir des spectres Raman et des paramètres quantitatifs de morphologie dudit agent microbien,
- déterminer la résistance aux agents anti-infectieux de l'agent microbien à partir des spectres Raman et des paramètres quantitatifs de morphologie dudit agent microbien.

2. Procédé selon la revendication 1, lesdites au moins deux conditions différentes en termes d'agent anti-infectieux étant deux concentrations différentes dudit agent anti-infectieux et/ou deux durées d'action différentes dudit agent anti-infectieux.

3. Procédé selon l'une des revendications 1 ou 2, comprenant les étapes consistant à :
- déterminer les spectres Raman de l'agent microbien en soumettant l'échantillon, à au moins deux moments durant la mise en culture dudit échantillon, à une spectroscopie Raman,
- déterminer les paramètres quantitatifs de morphologie de l'agent microbien en soumettant l'échantillon, à au moins deux moments durant la mise en culture dudit échantillon, à une détection optique,
- identifier l'agent microbien à partir des spectres Raman et des paramètres quantitatifs de morphologie dudit agent microbien,
- déterminer la résistance aux agents anti-infectieux de l'agent microbien à partir des spectres Raman et des paramètres quantitatifs de morphologie dudit agent microbien.

4. Procédé selon l'une des revendications précédentes, ladite détection optique étant réalisée par microscopie, par détection holographique ou par diffusion dynamique de la lumière, ladite microcopie étant de la microscopie optique ou de la microscopie à fluorescence.

5. Procédé selon l'une des revendications précédentes, comprenant en outre l'étape consistant à :
- déterminer le croissance cellulaire dudit agent microbien par détection optique;
la détermination de la résistance aux agents anti-infectieux étant basées sur les spectres de Raman déterminés, les paramètres quantitatifs de morphologie déterminés et la croissance cellulaire déterminée chez ledit agent microbien.

6. Procédé selon l'une des revendications précédentes, ledit au moins un agent microbien étant une bactérie et ledit agent anti-infectieux étant un agent antibiotique.

7. Procédé selon l'une des revendications précédentes, la détermination de la résistance aux agents anti-infectieux de l'agent microbien comprenant l'utilisation d'un premier indicateur de la résistance dudit agent microbien audit agent anti-infectieux, le premier indicateur de la résistance dudit agent microbien audit agent anti-infectieux étant une morphologie de l'agent microbien qui est inchangée par rapport aux données d'un contrôle négatif.

8. Procédé selon la revendication 7, la détermination de la résistance aux agents anti-infectieux de l'agent microbien comprenant l'utilisation d'un deuxième indicateur de la résistance dudit agent microbien audit agent anti-infectieux, le deuxième indicateur de la résistance dudit agent microbien audit agent anti-infectieux étant un spectre Raman de l'agent microbien qui, par rapport aux données d'un contrôle négatif, est inchangé ou qui a subi des modifications caractéristiques d'un certain mécanisme de résistance et/ou la détermination de la résistance aux agents anti-infectieux de l'agent microbien comprenant l'utilisation d'un troisième indicateur de la résistance dudit agent microbien audit agent anti-infectieux, le troisième indicateur de la résistance dudit agent microbien audit agent anti-infectieux étant une croissance cellulaire de l'agent microbien qui est inchangée par rapport aux données d'un contrôle négatif.

9. Procédé selon les revendications 7 ou 8, les données d'un contrôle négatif étant celles d'un échantillon qui comprend ledit agent microbien sans aucun agent anti-infectieux.

10. Procédé selon l'une des revendications précédentes, ledit procédé étant en outre mis en œuvre avec un échantillon servant de contrôle et contenant ledit au moins un agent microbien sans aucun agent anti-infectieux et/ou la durée dudit procédé étant inférieure à 180 minutes, de préférence inférieure à 150 minutes, de préférence encore inférieure à 120 minutes, avec la plus grande préférence inférieure à 60 minutes.

11. Procédé selon l'une des revendications précédentes, ledit échantillon ayant été prélevé chez un individu, s'agissant de préférence d'un mammifère, avec une préférence particulière d'un être humain, et/ou contenant 1*10² à 1*10⁵, de préférence 5*10³ à 1*10⁴ agents microbiens.

12. Procédé selon l'une des revendications précédentes, comprenant la génération d'un champ électrique non-homogène au sein de l'échantillon.
